# EUROPEAN PATENT SPECIFICATION

(11) **EP 4 041 286 B1**
(45) Date of publication and mention of the grant of the patent: **15.07.2026**
(21) Application number: 20819907.5
(22) Date of filing: 13.10.2020
(51) Int. Cl.: A61K 38/46, A61P 31/04, C07K 14/31, C12N 1/06

(54) **RECOMBINANT POLYPEPTIDE FOR USE AS A MEDICINE, ANTISEPTIC AGENT, ANTIBACTERIAL AGENT, ANTI-INFLAMMATORY AGENT, COMPOSITIONS COMPRISING IT AND USES THEREOF**
REKOMBINANTES POLYPEPTID ZUR VERWENDUNG ALS MEDIZIN, ANTISEPTISCHES MITTEL, ANTIBAKTERIELLES MITTEL, ENTZÜNDUNGSHEMMENDES MITTEL, ZUSAMMENSETZUNGEN DAMIT UND VERWENDUNGEN DAVON
POLYPEPTIDE RECOMBINANT DESTINÉ À ÊTRE UTILISÉ EN TANT QUE MÉDICAMENT, AGENT ANTISEPTIQUE, AGENT ANTIBACTÉRIEN, AGENT ANTI-INFLAMMATOIRE, COMPOSITIONS LE COMPRENANT ET LEURS UTILISATIONS

(30) Priority: 13.10.2019 PL 43144519
(43) Date of publication of application: 17.08.2022
(73) Proprietor: Miedzynarodowy Instytut Biologii Molekularnej I Komorkowej W Warszawie, 02-109 Warszawa (PL)
(72) Inventor: SABALA, Izabela, 96-100 Skierniewice (PL); JAGIELSKA, Elzbieta, 00-127 Warszawa (PL); KUCZYNSKA, Beata, 00-034 Warszawa (PL); AUGUSTYNIAK, Weronika, 08-330 Kosów Lacki (PL); MITKOWSKI, Pawel, 05-120 Legionowo (PL); WYSOCKA, Alicja, 01-592 Warszawa (PL)
(74) Representative: Grzelak, Anna
(86) International application number: PCT/PL2020/050075
(87) International publication number: WO 2021/075988

(56) References cited:
- WO-A2-2007/130655
- US-A1- 2011 027 249
- ODINTSOV S G ET AL: "Latent LytM at 1.3A Resolution", JOURNAL OF MOLECULAR BIOLOGY, ACADEMIC PRESS, UNITED KINGDOM, vol. 335, no. 3, 16 January 2004 (2004-01-16), pages 775 - 785, XP004480547, ISSN: 0022-2836, DOI: 10.1016/J.JMB.2003.11.009
- RODRIGUEZ-RUBIO LORENA ET AL: "Enhanced Staphylolytic Activity of the Staphylococcus aureus Bacteriophage vB SauS-phiIPLA88 HydH5 Virion-Associated Peptidoglycan Hydrolase: Fusions, Deletions, and Synergy with LysH5", APPLIED AND ENVIRONMENTAL MICROBIOLOGY, AMERICAN SOCIETY FOR MICROBIOLOGY, US, vol. 78, no. 7, 1 April 2012 (2012-04-01), pages 2241 - 2248, XP009162655, ISSN: 0099-2240, DOI: 10.1128/AEM.07621-11
- VERBREE CAROLIN T. ET AL: "Corrected and Republished from: Identification of Peptidoglycan Hydrolase Constructs with Synergistic Staphylolytic Activity in Cow's Milk", APPLIED AND ENVIRONMENTAL MICROBIOLOGY, vol. 84, no. 1, 1 January 2018 (2018-01-01), US, XP055881646, ISSN: 0099-2240, Retrieved from the Internet <URL:https://journals.asm.org/doi/pdf/10.1128/AEM.02134-17> DOI: 10.1128/AEM.02134-17
- RODR�GUEZ-RUBIO LORENA ET AL: "Potential of the Virion-Associated Peptidoglycan Hydrolase HydH5 and Its Derivative Fusion Proteins in Milk Biopreservation", PLOS ONE, vol. 8, no. 1, 24 January 2013 (2013-01-24), pages e54828, XP055881670, Retrieved from the Internet <URL:https://journals.plos.org/plosone/article/file?id=10.1371/journal.pone.0054828&type=printable> DOI: 10.1371/journal.pone.0054828
- DANIEL C. OSIPOVITCH ET AL: "Fusion with a cell wall binding domain renders autolysin LytM a potent anti-Staphylococcus aureus agent", FEMS MICROBIOLOGY LETTERS, vol. 362, no. 2, 8 December 2014 (2014-12-08), pages 1 - 7, XP055769746, DOI: 10.1093/femsle/fnu035
- ELZBIETA JAGIELSKA ET AL: "LytM Fusion with SH3b-Like Domain Expands Its Activity to Physiological Conditions", MICROBIAL DRUG RESISTANCE., vol. 22, no. 6, 1 September 2016 (2016-09-01), US, pages 461 - 469, XP055440506, ISSN: 1076-6294, DOI: 10.1089/mdr.2016.0053
- ELZBIETA JAGIELSKA ET AL: "Figure S4", MICROBIAL DRUG RESISTANCE., vol. 22, no. 6, 1 September 2016 (2016-09-01), US, pages 1, XP055769772, ISSN: 1076-6294, DOI: 10.1089/mdr.2016.0053
- ELZBIETA JAGIELSKA ET AL: "Supplementary figure S5", MICROBIAL DRUG RESISTANCE., vol. 22, no. 6, 1 September 2016 (2016-09-01), US, pages 1, XP055769776, ISSN: 1076-6294, DOI: 10.1089/mdr.2016.0053
- HOERNIG K J ET AL: "Evaluation of a lysostaphin-fusion protein as a dry-cow therapy forStaphylococcus aureusmastitis in dairy cattle", JOURNAL OF DAIRY SCIENCE, vol. 99, no. 6, June 2016 (2016-06-01), pages 4638 - 4646, XP029547718, ISSN: 0022-0302, DOI: 10.3168/JDS.2015-10783
- BHAGWAT AMALA ET AL: "Opportunities for broadening the application of cell wall lytic enzymes", APPLIED MICROBIOLOGY AND BIOTECHNOLOGY, SPRINGER BERLIN HEIDELBERG, BERLIN/HEIDELBERG, vol. 104, no. 21, 18 September 2020 (2020-09-18), pages 9019 - 9040, XP037271285, ISSN: 0175-7598, [retrieved on 20200918], DOI: 10.1007/S00253-020-10862-Y

## Description

### FIELD OF THE INVENTION

The present invention relates to a a recombinant polypeptide comprising(i) a LytM catalytic domain (LytMCD), comprising an amino acid sequence shown in SEQ ID NO: 1 or having at least 80% identity thereto, which retains the bacteriolytic activity against Staphylococcus aureus and wherein the zinc-coordinating amino acids in the active center are not changed and are His in position 26, Asp in position 30, His in position 109 of the SEQ ID NO: 1;(ii) a lysostaphin domain, binding the cell wall of bacteria (LssCWT), comprising an amino acid sequence shown in SEQ ID NO: 2 or having at least 80% identity thereto;(iii) from the LssCWT domain side, at the N or C terminus of the polypeptide, has an attached Cell-Penetrating Peptide (CPP) signal sequence directing to the inside of an eukaryotic cell;for use in the treatment of a disease and/or condition caused by a bacterium of the genus Staphylococcus. The invention also includes a pharmaceutical or veterinary composition comprising the recombinant polypeptide for use in the treatment of a disease and /or condition caused by a bacterium of the genus *Staphylococcus.*

The invention includes a use of the recombinant polypeptide as disinfectant, *in-vitro* antibacterial agent, *in-vitro* active agent in a cosmetic or care composition for cosmetic, care and hygiene use in humans and animals against a bacterium of the genus *Staphylococcus.* The invention also relates to a cosmetic or care composition for cosmetic, care and hygienic use in humans or animals comprising the recombinant polypeptide, wherein the recombinant polypeptide is used to limit the occurrence or to remove a bacteria of the genus *Staphylococcus.*

The invention also relates to a recombinant polypeptide comprising(i) a LytM catalytic domain (LytMCD), comprising an amino acid sequence shown in SEQ ID NO: 1 or having at least 80% identity thereto which retains the bacteriolytic activity against Staphylococcus aureus, and wherein the zinc-coordinating amino acids in the active center are not changed and are His in position 26, Asp in position 30, His in position 109 of the SEQ ID NO: 1;(ii) a lysostaphin domain, binding the cell wall of bacteria (LssCWT), comprising an amino acid sequence shown in SEQ ID NO: 2 or having at least 80% identity thereto, wherein the recombinant polypeptide exhibits bacteriolytic activity in raw milk and/or serum, for use in the treatment of mastitis or prevention of developing mastitis in the cow and as *in-vitro* antibacterial agent against a bacterium of the genus *Staphylococcus* in raw milk.

### STATE OF THE ART

Bacteria of the genus *Staphylococcus* (staphylococci) are responsible for a whole range of infections in humans and animals. Moreover, many *Staphylococcus* species are resistant to antibiotics; it is estimated that 30-50% of humans are carriers of these bacteria. Therefore, it is urgently required to provide alternative methods of treatment of infections caused by bacteria of the genus *Staphylococcus.* Even though antibiotics are still major therapeutic agents for the treatment of staphylococcal infections, they often lead to considerable side effects and further contribute to the emergence of antibiotic resistance. Therefore, new, alternative and effective methods of treatment of infections caused by staphylococci are urgently needed.

Dermatological and inflammatory disorders may be caused by various species of bacteria, including staphylococci. They can occur spontaneously or, e.g., in a wound, ulcer or burn. Infected wounds can be life-threatening. Additionally, biofilm producing bacteria can be present in the infected wounds, or wounds can be secondarily infected with bacteria (Bowler et al., 2001). Infections with antibiotic-resistant *Staphylococcus aureus* strains, such as MRSA (methicillin-resistant *Staphylococcus aureus*) strains are particularly dangerous. Staphylococcal infections are particularly common in the case of diabetic foot, pressure ulcers and burns.

*Staphylococcus* is also one of the causative agents of udder inflammation in dairy cows, known as mastitis. Mastitis is a persistent, inflammatory reaction of the mammary gland and udder tissue, and is a major endemic disease of dairy cattle, leading to annual losses of more than 1 billion Euro in Europe. Mastitis arises when leukocytes are released into the mammary gland, as an immune response to bacterial invasion of the teat canal by different bacteria. Milk-secreting tissues and ducts of the mammary glands are damaged by toxins released by bacteria. Published data show that more than half of the bacteria isolated from udders are *Staphylococci* (Malinowski and Smulski, 2007). *S. aureus* is responsible for both acute clinical and sub-clinical forms of mastitis, and particularly chronic infections. In cases of acute and chronic udder inflammation, especially caused by *S. aureus,* antibiotics are the standard-of-care treatment.

The infected cows are carriers of pathogenic bacteria, therefore several infected cows in a herd can form a persistent reservoir for future outbreaks of staphylococcal infections. In particular, *S*. *aureus* is rapidly transferred between the animals. Mastitis poses a serious threat to human health as well. Bacterial infections of milk and its products may cause food poisoning in humans who consume them.

One of the possible approaches to combat mastitis is the use of bacteriolytic enzymes. Although various bacteriolytic enzymes and chimeric versions thereof have been recently tested, none of them demonstrated a satisfactory enzymatic activity in raw cow's milk (see **Table 1** below). Chimeras composed of the LytM catalytic domain and the lysostaphin cell wall binding domain have been described earlier (Osipovitch and Griswold, 2015, Jagielska, Chojnacka et al., 2016), however, their activity in milk and serum was not demonstrated at that time.

This property is very rare and cannot be predicted based on the properties of individual domains that make up the chimera. Although many phage lysins and chimeric enzymes have been tested, even those that showed activity in physiological conditions and pasteurized milk, did not eliminate staphylococci from raw milk (Verbree, Datwyler et al., 2018, Rodriguez-Rubio, Martinez et al., 2013) and/or they lost their bacteriolytic properties in the presence of serum, which eliminates their practical application in veterinary medicine and medicine (Osipovitch, Therrien et al., 2015).

From the publication WO2012144912, a stable LytMCD derivative (LytM₁₈₅₋₃₁₆) from *Staphylococcus aureus* is known, with the activity of glycylglycine endopeptidase against *S. aureus* bacteria under non-physiological conditions, at very low conductivity, below 10 mS/cm, low salt concentration and low temperature. This derivative of LytM185-316 lacks a bacterial cell wall binding domain and is also inactive under physiological conditions in in vivo staphylococcal infections such as mastitis or a staphylococcal wound.

From the publication WO2007/130655, a chimeric TAME polypeptide, which includes a cell wall lysing domain LytM and a lysostaphin cell wall binding domain is known, however, the chimera presented in sequence 9, in the light of the available information, cannot exhibit biological activity, as it does not include zinc coordinating amino acids in the active center, necessary for the activity of the LytM enzyme.

The chimeras presented in that publication do not show activity against *Staphylococcus,* in particular, *Staphylococcus aureus* under physiological conditions, which is whole milk, and are not able to act as a medicine, antibacterial agent and/or anti-inflammatory agent, which prevents or treats dermatological and/or inflammatory disorders, particularly those caused by bacteria of the genus *Staphylococcus,* in particular *Staphylococcus aureus,* particularly in an animal suffering from mastitis or at risk of developing such an inflammation.

### SUMMARY OF THE INVENTION

The recombinant polypeptide and the veterinary composition for use according to the present invention differ from the standard antibiotics in their specificity and activity against *Staphylococcus* strains. Unexpectedly, it was found that by combining the LytM₁₈₅₋₃₁₆ catalytic domain with the lysostaphin cell wall binding domain (LssCWT), preferably via a peptide linker (Lss_linker), and adding a Cell-Penetrating Peptide (CPP), a recombinant polypeptide with strong antibacterial properties, especially against staphylococci, active under physiological conditions is obtained. The inventors of the present invention unexpectedly found that the recombinant polypeptide for use according to the invention is highly active in raw cow's milk, preferably in raw undiluted milk, and in serum, as well as in wounds infected by staphylococci. Moreover, an unexpectedly rapid and effective bacteriolytic activity against *Staphylococcus* strains was observed. This is a valuable property, considering the efficacy provided by the polypeptide for use according to the invention, and pharmaceutical compositions comprising thereof. Unlike most antibiotics, the recombinant polypeptide for use according to the invention does not require any metabolic activity of bacteria or bacterial growth to be effective, as it acts bacteriolytically upon contact with the bacterial cell wall. This property makes the compositions comprising the recombinant polypeptide, particularly the veterinary composition for use according to the present invention, suitable for quick reduction of bacterial count in the infected hosts. The inventors unexpectedly found that due to the above properties, the recombinant polypeptide and compositions comprising thereof, particularly the veterinary composition, can also solve the problem of antibiotic resistance in staphylococcal infections. Moreover, the inventors unexpectedly found that the recombinant polypeptide for use according to the invention is highly specific for *Staphylococcus* species and does not cause lysis of non-target bacteria, including commensal bacteria, thereby potentially reducing side effects.

In **Table 1** below, a summary of the activities of the various peptidoglycan hydrolases is shown.

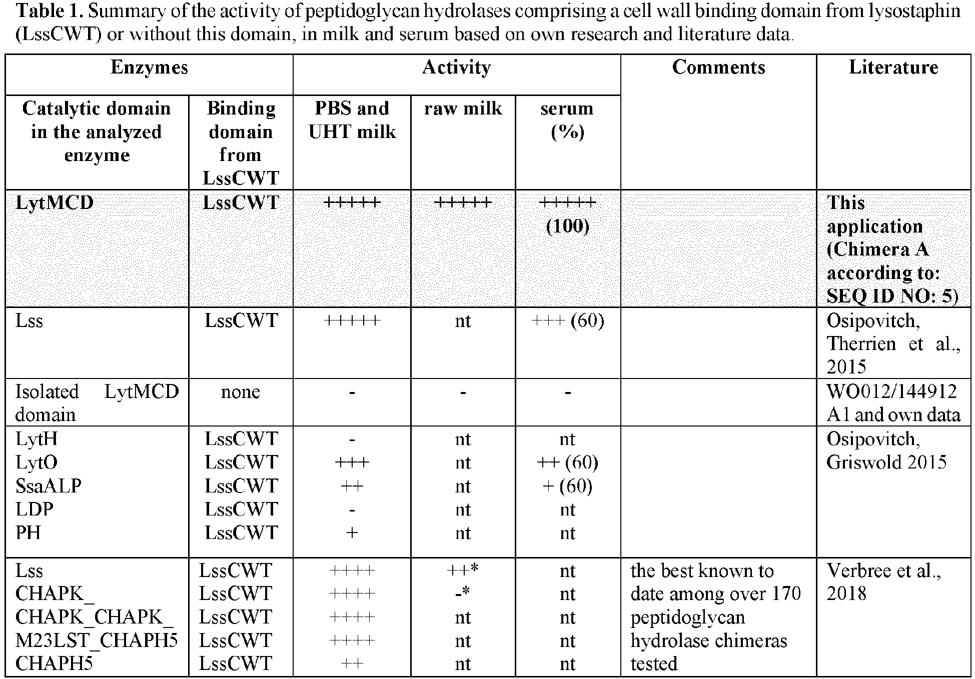

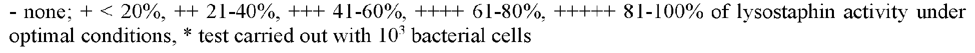

The are described recombinant polypeptides and compositions comprising thereof, for use as disinfectant, antiseptic agent, medicine, *in-vitro* antibacterial agent and/or anti-inflammatory agent, which prevents or treats dermatological and/or inflammatory disorders, antibacterial agent, as well as an active agent in cosmetic, care or hygienic compositions for use for humans and animals. In particular, the inventors have found that the recombinant polypeptides are effective in treating mastitis in ruminants, in particular, dairy cows. Experimental data provided below prove the recombinant polypeptide to be effective in treating mastitis in ruminants by effectively binding to the outer surface of bacteria of the genus *Staphylococcus,* in particular *Staphylococcus aureus,* causing cell lysis and thereby eliminating the bacteria.

The inventors have further shown *in vivo* that the recombinant polypeptides and compositions comprising them are effective in combating infections with bacteria of the genus *Staphylococcus,* in particular *Staphylococcus aureus,* particularly skin infections and infected wounds, particularly in the topical treatment of dermatological diseases, wounds and conditions of skin and/or soft tissues. One aspect of the invention relates to a recombinant polypeptide comprising (i) a LytM catalytic domain (LytMCD), comprising an amino acid sequence shown in SEQ ID NO: 1 or having at least 80% identity thereto, which retains the bacteriolytic activity against *Staphylococcus aureus* and wherein the zinc-coordinating amino acids in the active center are not changed and are His in position 26, Asp in position 30, His in position 109 of the SEQ ID NO: 1; (ii) a lysostaphin cell wall binding domain, binding the cell wall of bacteria (LssCWT), comprising an amino acid sequence shown in SEQ ID NO: 2 or having at least 80% identity thereto, and (iii) from the LssCWT domain side, at the N or C terminus of the polypeptide, has an attached Cell-Penetrating Peptide (CPP) signal sequence directing to the inside of an eukaryotic cell, (iV) optionally, a peptide linker, linking the LytM catalytic domain and the lysostaphin cell wall binding domain, for use in the treatment of a disease and/or condition caused by a bacterium of the genus *Staphylococcus..*

In the preferred recombinant polypeptide with CPP for the abovementioned use, the LytM catalytic domain consists of the amino acid sequence shown in SEQ ID NO: 1.

In the preferred recombinant polypeptide with CPP for the abovementioned use, the lysostaphin cell wall binding domain consists of the amino acid sequence shown in SEQ ID NO: 2.

In the preferred recombinant polypeptide with CPP for the abovementioned use, the peptide linker, linking the LytM catalytic domain and the lysostaphin cell wall binding domain, comprises the amino acid sequence shown in SEQ ID NO: 3 or SEQ ID NO: 4, preferably, it consists of the sequence SEQ ID NO: 3.

The recombinant polypeptide with CPP for the abovementioned use, preferably comprises the sequence SEQ ID NO: 5 or is encoded by a nucleotide sequence comprising SEQ ID NO: 6.

The recombinant polypeptide with CPP for the abovementioned use, preferably comprises the sequence SEQ ID NO: 7 or is encoded by a nucleotide sequence comprising SEQ ID NO: 8.

The recombinant polypeptide with CPP for the abovementioned use, preferably comprises the signal sequence is selected from SEQ ID NO: 9-19.

The recombinant polypeptide with CPP for the abovementioned use, is preferably used n the treatment of a disease and/or condition caused by *Staphylococcus aureus.*

The recombinant polypeptide with CPP for the abovementioned use, is preferably used in treatment of a disease and/or condition selected from the group consisting of acne, pimples, dermatitis, preferably atopic dermatitis, infections and/or injuries of a mucous membrane or the skin, wounds, diabetic foot, ulcers, burns and an inflammation of the mammary gland, udder, eye, ear, throat, sinuses or genitals.

The recombinant polypeptide with CPP for the abovementioned use, is preferably formulated for topical administration, preferably in the form of a cream, lotion, solution, ointment, gel, foam, transdermal patch, powder, paste, suspension, tape, pad, swab, wound dressing or compress.

The recombinant polypeptide with CPP for the abovementioned use, is preferably formulated for intramammary (including intra-nipple/teat/udder) delivery.

The recombinant polypeptide with CPP for the abovementioned use, is preferably delivered to a nipple or teat, to udder, by infusion, rinsing or dipping.

The recombinant polypeptide with CPP for the abovementioned use, is preferably delivered to a female human or non-human animal suffering from mastitis or at risk of developing mastitis, preferably a ruminant, more preferably a dairy cow.

In some embodiments, the recombinant polypeptide with CPP is used in the in the treatment of a disease and/or condition caused by a bacterium of the genus *Staphylococcus* which are dermatological and/or inflammatory disorder, wherein the dermatological disorder is selected from the group consisting of acne, pimples, dermatitis, preferably atopic dermatitis, infections and/or injuries of a mucous membrane, skin, wounds, diabetic foot, ulcers, burns and an inflammation of the mammary gland, udder, eye, ear, throat, sinuses or genitals.

Preferably, the recombinant polypeptide for use exhibits bacteriolytic activity in raw milk and/or serum, wherein the raw milk is preferably raw cow's milk, and wherein the serum is preferably bovine or human serum. Thus, the recombinant polypeptide for use according to the invention, is preferably contacted with the bacteria to be eliminated from milk, most preferably raw milk, or from serum or blood or from eukaryotic cells.

In some embodiments, the recombinant polypeptide with CPP according to the present invention may be formulated for topical administration. Preferably, the formulation is in the form of a cream, lotion, solution, ointment, gel, foam, transdermal patch, powder, paste, tincture, tape, pad, swab, wound dressing or compress.

In some embodiments, the recombinant polypeptide with CPP is formulated for intramammary delivery.

In some embodiments, the recombinant polypeptide with CPP is delivered to a nipple or teat, preferably intramammary (including ), preferably by infusion, rinsing or dipping.

The preferred embodiments relate to the delivery of the recombinant polypeptide with CPP to a female human or non-human animal suffering from mastitis or at risk of developing mastitis, preferably a ruminant, more preferably a dairy cow.

Another aspect of the invention relates to a pharmaceutical or veterinary composition comprising a recombinant polypeptide, comprising (i) a LytM catalytic domain (LytMCD), comprising an amino acid sequence shown in SEQ ID NO: 1 or having at least 80% identity thereto, which retains the bacteriolytic activity against *Staphylococcus aureus,* and wherein the zinc-coordinating amino acids in the active center are not changed and are His in position 26, Asp in position 30, His in position 109 of the SEQ ID NO: 1, (ii) a lysostaphin domain, binding the cell wall of bacteria (LssCWT), comprising an amino acid sequence shown in SEQ ID NO: 2 or having at least 80% identity thereto, (iii) from the LssCWT domain side, at the N or C terminus of the polypeptide, has an attached Cell-Penetrating Peptide (CPP) signal sequence directing to the inside of an eukaryotic cell, and (iV) optionally, a peptide linker, linking the LytM catalytic domain and the lysostaphin cell wall binding domain, and a pharmaceutically or veterinary acceptable excipient, for use in the treatment of a disease and /or condition caused by a bacterium of the genus *Staphylococcus.*

In the preferred pharmaceutical or veterinary composition for the abovementioned use, in a recombinant polypeptide with CPP, the LytM catalytic domain consists of the amino acid sequence shown in SEQ ID NO: 1.

In the preferred pharmaceutical or veterinary composition for the abovementioned use, in a recombinant polypeptide with CPP, the lysostaphin cell wall binding domain consists of the amino acid sequence shown in SEQ ID NO: 2.

In the preferred pharmaceutical or veterinary composition for the abovementioned use, in a recombinant polypeptide with CPP, the peptide linker, linking the LytM catalytic domain and the lysostaphin cell wall binding domain, comprises the amino acid sequence shown in SEQ ID NO: 3 or SEQ ID NO: 4, preferably, it consists of the sequence SEQ ID NO: 3.

In the preferred pharmaceutical or veterinary composition for the abovementioned use, a recombinant polypeptide with CPP comprises the sequence SEQ ID NO: 5 or is encoded by a nucleotide sequence comprising SEQ ID NO: 6.

In the preferred veterinary composition for the abovementioned use, a recombinant polypeptide comprises the sequence SEQ ID NO: 7 or is encoded by a nucleotide sequence comprising SEQ ID NO: 8, preferably.

In the preferred pharmaceutical or veterinary composition for the abovementioned use, a recombinant polypeptide with CPP, the attached CCP signal sequence directing to the inside of an eukaryotic cell is selected from SEQ ID NO: 9-19.

The pharmaceutical or veterinary composition for the abovementioned use, is preferably used in a dermatological disorder and/or inflammation, which is selected from the group consisting of dermatitis, injuries and/or infections of a mucous membrane or of the skin, wounds, ulcers, and inflammation of the mammary gland, inflammation of an eye, ear, throat, sinuses or genitals.

The pharmaceutical or veterinary composition for the abovementioned use, is preferably formulated for intramammary delivery.

The pharmaceutical or veterinary composition for the abovementioned use, is preferably used in the treatment of mastitis in ruminants.

Specific embodiments relate to the pharmaceutical or veterinary composition for use in the treatment of mastitis in ruminants.

The veterinary composition for use, is described for use in dermatological disorder and/or inflammation selected from the group consisting of dermatitis, injuries and/or infections of a mucous membrane or of the skin, wounds and inflammation of the udder, eye, ear, throat, sinuses or genitals.

The veterinary composition may be intended for intramuscular administration.

In yet another embodiment, the pharmaceutical or veterinary composition is intended for use in the treatment of mastitis in ruminants.

Preferably, the veterinary or other pharmaceutical composition for use comprising the recombinant polypeptide with CPP according to the invention is contacted with the bacteria to be eliminated from milk, most preferably from raw milk, or from serum or blood.

The invention also relates to the use of a recombinant polypeptide comprising (i) a LytM catalytic domain (LytMCD), comprising an amino acid sequence shown in SEQ ID NO: 1 or having at least 80% identity thereto, which retains the bacteriolytic activity against *Staphylococcus aureus,* and wherein the zinc-coordinating amino acids in the active center are not changed and are His in position 26, Asp in position 30, His in position 109 of the SEQ ID NO: 1, (ii) a lysostaphin domain, binding the cell wall of bacteria (LssCWT), comprising an amino acid sequence shown in SEQ ID NO: 2 or having at least 80% identity thereto, (iii) from the LssCWT domain side, at the N or C terminus of the polypeptide, has an attached Cell-Penetrating Peptide (CPP) signal sequence directing to the inside of an eukaryotic cell, and (iv) optionally, a peptide linker, linking the LytM catalytic domain and the lysostaphin cell wall binding domain, as disinfectant, *in-vitro* antibacterial agent, *in-vitro* active agent in a cosmetic, care or hygienic composition for humans or animals against a bacterium of the genus *Staphylococcus..*

In the preferred use of the recombinant polypeptide with CPP, the LytM catalytic domain consists of the amino acid sequence shown in SEQ ID NO: 1.

In the preferred use of the recombinant polypeptide with CPP, the lysostaphin cell wall binding domain consists of the amino acid sequence shown in SEQ ID NO: 2.

In the preferred use of the recombinant polypeptide with CPP, the peptide linker, linking the LytM catalytic domain and the lysostaphin cell wall binding domain, comprises the amino acid sequence shown in SEQ ID NO: 3 or SEQ ID NO: 4, preferably, it consists of the sequence SEQ ID NO: 3.

In the preferred use of the recombinant polypeptide with CPP, it comprises the sequence SEQ ID NO: 5 or is encoded by a nucleotide sequence comprising SEQ ID NO: 6.

In the preferred use of the recombinant polypeptide with CPP, it comprises the sequence SEQ ID NO: 7, or is encoded by a nucleotide sequence comprising SEQ ID NO: 8.

In the preferred use of the recombinant polypeptide with CPP, the signal sequence is selected from SEQ ID NO: 9-19.

In the preferred use of the recombinant polypeptide with CPP, when is used as a disinfectant, it is used for disinfecting surfaces, appliances and equipment, furniture, floors, particularly in hospitals, medical and dental offices, kitchens and bathrooms for disinfecting surfaces from *Staphylococcus,* preferably *Staphylococcus aureus.*

In the preferred use of the recombinant polypeptide with CPP, when the polypeptide is used as an *in-vitro* antibacterial agent, it is preferably used against *Staphylococcus aureus.*

In the preferred use of the recombinant polypeptide with CPP, it is used as *in-vitro* antibacterial agent in milk, serum, preferably in whole/raw milk.

In the preferred use of the recombinant polypeptide with CPP, when the polypeptide is used as *in-vitro* active agent in a cosmetic, care or hygienic composition for humans or animals, it is used as *in-vitro* active agent preferably against *Staphylococcus aureus.*

The invention also relates to a cosmetic or care composition for cosmetic, care and hygienic use in humans or animals, which comprises a recombinant polypeptide comprising (i) a LytM catalytic domain (LytMCD), comprising an amino acid sequence shown in SEQ ID NO: 1 or having at least 80% identity thereto, and wherein the zinc-coordinating amino acids in the active center are not changed and are His in position 26, Asp in position 30, His in position 109 of the SEQ ID NO: 1, (ii) a lysostaphin domain, binding the cell wall of bacteria (LssCWT), comprising an amino acid sequence shown in SEQ ID NO: 2 or having at least 80% identity thereto, (iii) from the LssCWT domain side, at the N or C terminus of the polypeptide, has an attached Cell-Penetrating Peptide (CPP) signal sequence directing to the inside of an eukaryotic cell, and (iii) optionally, a peptide linker, linking the LytM catalytic domain and the lysostaphin cell wall binding domain, wherein the composition additionally comprises at least one carrier approved for care and hygienic applications in humans or animals, and is intended for external use, and wherein the recombinant polypeptide is use to limit the occurrence or to remove a bacteria of the genus *Staphylococcus* in the cosmetic or care composition.

Preferred is a cosmetic or care composition for use, in which, in the recombinant polypeptide, the LytM catalytic domain consists of the amino acid sequence shown in SEQ ID NO: 1, equally preferred, in which, in the recombinant polypeptide, the lysostaphin cell wall binding domain consists of the amino acid sequence shown in SEQ ID NO: 2, equally preferred is a composition, in which, in the recombinant polypeptide, the peptide linker, linking the LytM catalytic domain and the lysostaphin cell wall binding domain comprises the amino acid sequence shown in SEQ ID NO: 3 or SEQ ID NO: 4, preferably, it consists of the sequence SEQ ID NO: 3, equally preferred is a composition, in which the recombinant polypeptide comprises the sequence SEQ ID NO: 5 or is encoded by a nucleotide sequence comprising SEQ ID NO: 6, , equally preferred is a composition, in which the recombinant polypeptide comprises the sequence SEQ ID NO: 7 or is encoded by a nucleotide sequence comprising SEQ ID NO: 8.

The preferred cosmetic or care composition for use is used to limit the occurrence or to preferably remove bacteria *Staphylococcus aureus.*

The invention also relates to recombinant polypeptide comprising (i) a LytM catalytic domain (LytMCD), comprising an amino acid sequence shown in SEQ ID NO: 1 or having at least 80% identity thereto which retains the bacteriolytic activity against *Staphylococcus aureus,* and wherein the zinc-coordinating amino acids in the active center are not changed and are His in position 26, Asp in position 30, His in position 109 of the SEQ ID NO: 1; (ii) a lysostaphin domain, binding the cell wall of bacteria (LssCWT), comprising an amino acid sequence shown in SEQ ID NO: 2 or having at least 80% identity thereto, wherein the recombinant polypeptide exhibits bacteriolytic activity in raw milk and/or serum, for use in the treatment of mastitis or prevention of developing mastitis in the cow,
caused by a bacterium of the genus *Staphylococcus,* wherein the recombinant polypeptide is administered to the raw milk in udder of a cow.

The preferred recombinant polypeptide for use in the treatment of mastitis or prevention of developing mastitis in the cow further, from the LssCWT domain side, at the N or C terminus of the polypeptide, has an attached CPP signal sequence directing to the inside of an eukaryotic cell, preferably the signal sequence is selected from SEQ ID NO: 9-19.

The preferred recombinant polypeptide for use in the treatment of mastitis or prevention of developing mastitis in the cow, wherein it is in the form of pharmaceutical or veterinary composition additionally comprising pharmaceutically or veterinary acceptable excipient..

The recombinant polypeptide for use in the treatment of mastitis or prevention of developing mastitis in the cow wherein the administration is performed by infusion, rinsing or dipping.

The invention also related to use of the recombinant polypeptide comprising: (i) a LytM catalytic domain (LytMCD), comprising an amino acid sequence shown in SEQ ID NO: 1 or having at least 80% identity thereto which retains the bacteriolytic activity against *Staphylococcus aureus,* and wherein the zinc-coordinating amino acids in the active center are not changed and are His in position 26, Asp in position 30, His in position 109 of the SEQ ID NO: 1; (ii) a cell wall binding lysostaphin domain, binding the cell wall of bacteria (LssCWT), comprising an amino acid sequence shown in SEQ ID NO: 2 or having at least 80% identity thereto; as *in-vitro* antibacterial agent against a bacterium of the genus *Staphylococcus* in raw milk.

In the preferred recombinant polypeptide for use in the treatment of mastitis or prevention of developing mastitis according to invention and the use of recombinant polypeptide as *in-vitro* antibacterial agent according to invention the LytM catalytic domain consists of the amino acid sequence shown in SEQ ID NO: 1

In the preferred recombinant polypeptide for use in the treatment of mastitis or prevention of developing mastitis according to invention and the use of recombinant polypeptide as *in-vitro* antibacterial agent according to invention the lysostaphin cell wall binding domain consists of the amino acid sequence shown in SEQ ID NO: 2.

In the preferred recombinant polypeptide for use in the treatment of mastitis or prevention of developing mastitis according to invention and the use of recombinant polypeptide as *in-vitro* antibacterial agent according to invention it further comprises a peptide linker, linking the LytM catalytic domain and the lysostaphin cell wall binding domain, wherein the peptide linker preferably comprises the amino acid sequence shown in SEQ ID NO: 3 or SEQ ID NO: 4, preferably, peptide linker consists of the sequence SEQ ID NO: 3.

In the preferred recombinant polypeptide for use in the treatment of mastitis or prevention of developing mastitis according to invention and the use of recombinant polypeptide as *in-vitro* antibacterial agent according to invention the recombinant polypeptide comprises the sequence SEQ ID NO: 5, or is encoded by a nucleotide sequence comprising SEQ ID NO: 6, or comprises the sequence SEQ ID NO: 7, or is encoded by a nucleotide sequence comprising SEQ ID NO: 8.

In the preferred recombinant polypeptide for use in the treatment of mastitis or prevention of developing mastitis according to invention and the use of recombinant polypeptide as *in-vitro* antibacterial agent according to invention the bacteria of the genus *Staphylococcus* is *Staphylococcus aureus.*

### DESCRIPTION OF DRAWINGS

For a better understanding of the invention, it has been illustrated in the embodiments and in the accompanying figures, in which:

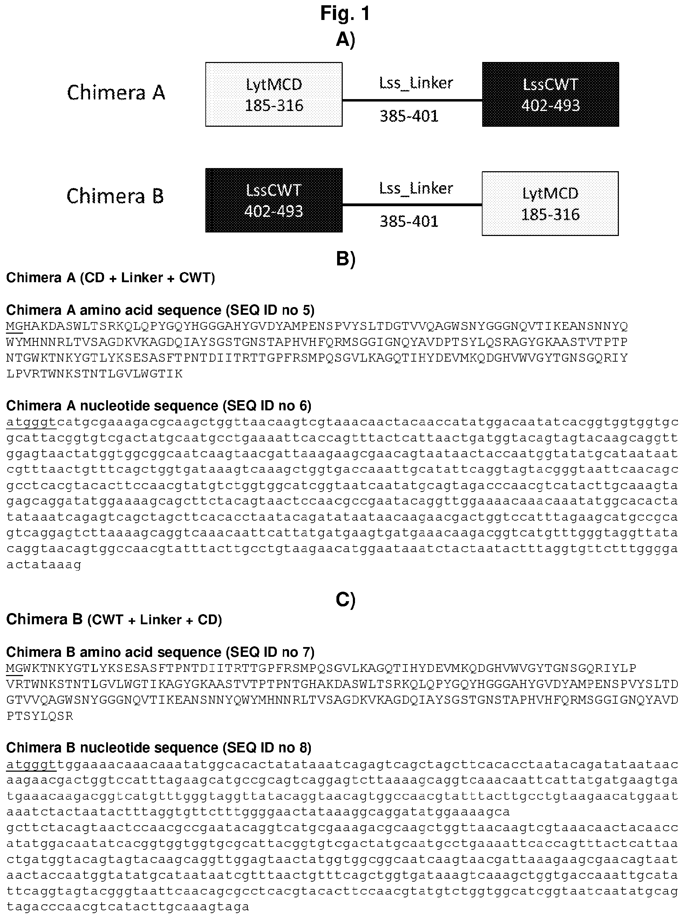

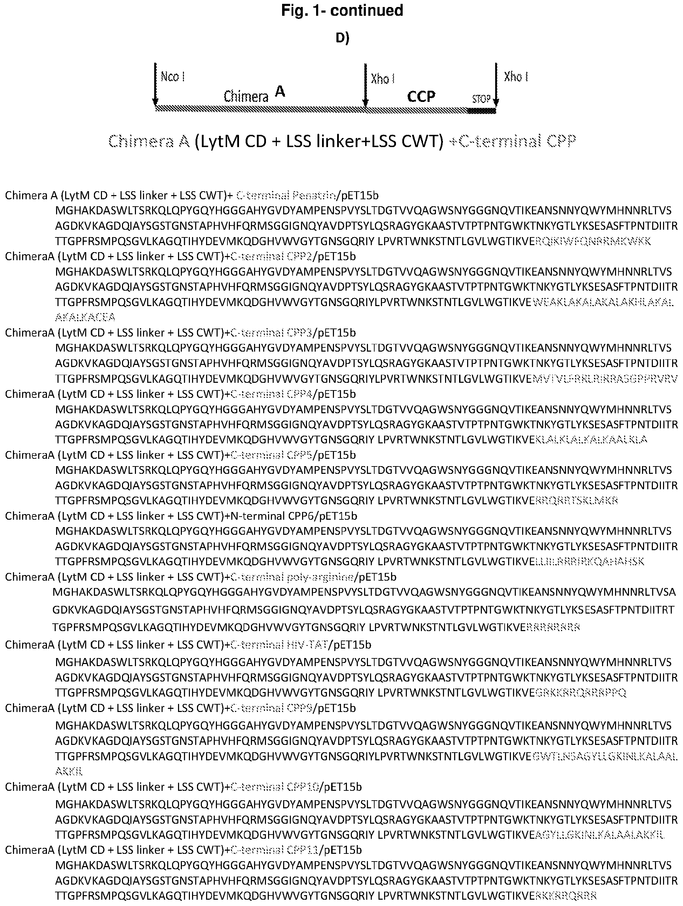

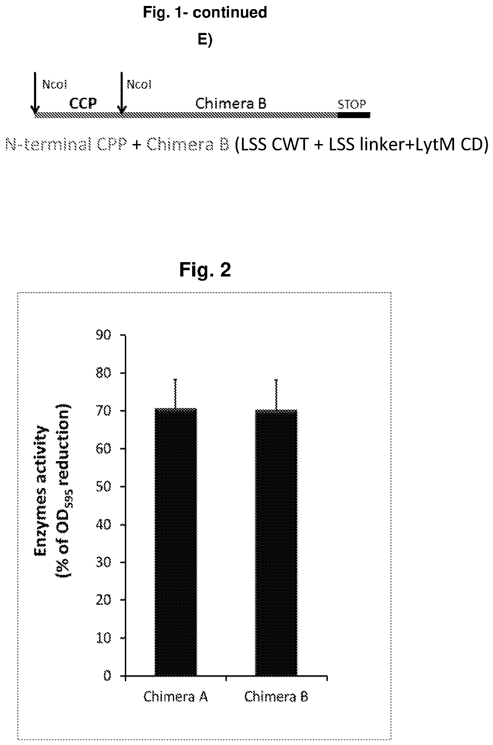

**FIG. 1****: A)** Schematic representation of domain organization in Chimeras A and B. Numbers in boxes represent amino acid positions in full-length proteins according to UniProt (https://www.uniprot.org/). LytMCD stands for the LytM catalytic domain (UniProt 033599), and LssCWT stands for the lysostaphin bacterial cell wall binding/targeting domain (Lss; UniProt P10547); **B)** shows the amino acid and nucleotide sequence of Chimera A, respectively; **C)** shows the amino acid and nucleotide sequence of Chimera B, respectively. Designations in B and C, respectively, underlined - sequences of the vector, light gray background - the CD domain of LytM, dark gray background - the CWT domain of Lss, unchecked - linker; **D)** schematic structure of Chimera A connected to the signal sequence at the C-terminus: Chimera A (LytM CD + LSS linker + LSS CWT)+C-terminal CPP and exemplary sequences of such chimeras; **E)** schematic structure of Chimera B connected to the signal sequence at the N-terminus: N-terminal CPP + Chimera B (LSS CWT + LSS linker+LytM CD), the signal sequences attached at the N-terminus can be, for example, the sequences shown in Table 1.
**FIG. 2****:** Comparison of the lytic activity of Chimeras A and B demonstrates comparable efficacy of these two enzymes.
**FIG. 3****:** Chimera A activity in UHT (ultra-heat temperature processing, ultra-heat treatment, or ultra-pasteurization) milk at 37 °C. Time-kill assay in milk spiked with 10³ CFU/ml of *S. aureus* cells (strain NCTC 8325-4) in the presence of 36 nM, 53 nM, 100 nM, 360 nM and 4000 nM of Chimera A Samples were incubated at 37 °C for 24 h. Negative control was carried out without the addition of the enzyme. The experiment was performed twice (mean ± SD).
**FIG. 4****:** 10³ CFU/ml of *S. aureus* (strain NCTC 8325-4) were inoculated into a UHT whole milk **(A)** and incubated for 1 hour at 37 °C. Chimera A caused complete elimination of bacteria in UHT whole milk **(B).**
**FIG. 5****:** Chimera A activity in UHT whole milk at 4 °C **(A)** and 22 °C **(B).** *S. aureus* cells (strain NCTC 8325-4; 10⁶ CFU/ml) were incubated with 100 nM of Chimera A for 24 hours. The negative control did not include the chimeric enzyme. The experiment was performed three times.

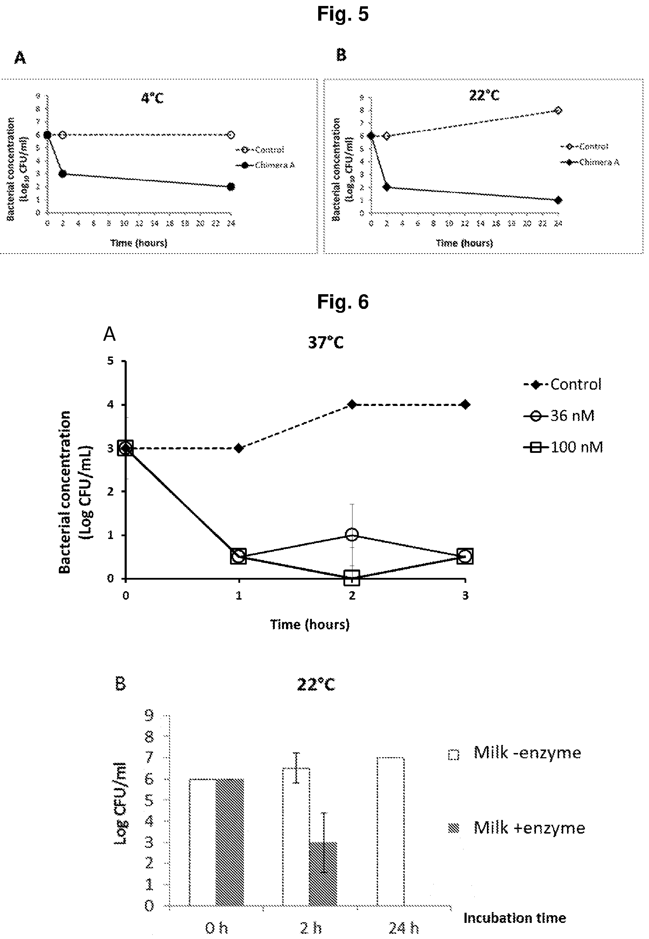

**FIG. 6****:** Chimera A activity in raw bovine milk (undiluted) at 37 °C. **A)** Time-kill assay in milk with the addition of 10³ CFU/ml of *S. aureus* cells (strain NCTC 8325-4) in the presence of either 36 nM or 100 nM of Chimera A. Samples were incubated at 37 °C for 3 hours; **B)** time-kill assay in milk with the addition of 10⁶ CFU/ml of *S. aureus* cells (strain NCTC 8325-4) and 100 nM of Chimera A. Samples were incubated at 22 °C for 24 hours, taking samples to assess the number of bacteria at time 0, after two hours and at the end of the experiment. In both the experiments, the negative control did not contain Chimera A. The experiments were performed twice (mean ± SD).
**FIG. 7****:** 10³ CFU/ml of *S. aureus* cells (strain NCTC 8325-4) were inoculated into raw, undiluted cow's milk and incubated for 1 hour at 37 °C with or without the addition of Chimera A. Assessment of staphylococcal cell number in the inoculated milk samples before the enzyme treatment **(A),** after 1 hour of incubation with Chimera A **(B),** after 1 hour of incubation without Chimera A **(C).**
**FIG. 8****:** Somatic cell count measured before and after 7 days of treatment with Chimera A.
**FIG. 9****:** Bacteriolytic activity of Chimera A in the presence of fetal bovine serum (FBS). The activity of 100 nM of Chimery A was estimated as a reduction of turbidity of bacterial cells suspension within 1 hour at room temperature.
**FIG. 10****.** Alignment of the amino acid sequence of Chimera A and the chimera known in the state of the art. Zinc-coordinating amino acids in the active center, necessary for integrity and activity of the enzyme, are highlighted in gray.
**FIG. 11****.** Comparison of the lytic activity of Chimera B, with and without the CPP sequence, against *S. aureus* under different environmental conditions: A- glycine buffer, B- bovine serum, C- UHT milk.
**FIG. 12****.** Antimicrobial effect of a hydrogel wound dressing, with or without a described Chimera, on the treatment of wounds infected by staphylococci. The graph shows the number of bacterial cells counted. Each dot represents one mouse; horizontal bar, calculated mean value of *S. aureus* in the isolates from infected wounds. Black circle - negative control, and white circle with a black outline-tested sample (results shown for Chimera A).
**FIG. 13****.** *In vivo* antimicrobial and anti-inflammatory effects of a hydrogel wound dressing with a recombinant polypeptide (Chimera A).

### DETAILED DESCRIPTION OF THE INVENTION

The following explanations and general definitions relate to the descriptions of preferred embodiments of the invention, given below.

The present invention as illustratively described in the following may suitably be practiced in the absence of any element or elements, limitation or limitations, not specifically disclosed herein.

The present invention will be described with respect to particular embodiments and with reference to certain figures, but the invention is not limited thereto, and is limited only by the claims.

When the term "comprising" or "including" is used in this description and claims, it does not exclude other elements. For the purposes of the present invention, the term "consisting of" is considered a preferred embodiment of the term "comprising", "including". If a group is defined below, which contains at least a certain number of embodiments, this should also be understood as disclosing a group, which preferably consists of only these embodiments.

The terms "about" or "approximately" in the context of the present invention stand for a range of accuracy that a person skilled in the art will understand to be sufficient to still provide the technical effect of the given feature. The term "typically" is to be understood as a deviation from the indicated numerical value of ± 10%, preferably ± 5%.

Technical terms are used by their typical meaning. If a specific meaning is conveyed to certain terms, the meaning will be described in detail in reference to the context in which the given term was used.

A recombinant polypeptide not forming part of the invention comprising (i) a LytM catalytic domain, comprising an amino acid sequence shown in SEQ ID NO: 1 or having at least 80% identity thereto, (ii) a lysostaphin cell wall binding domain, comprising an amino acid sequence shown in SEQ ID NO: 2 or having at least 80% identity thereto, and (iii) optionally, a peptide linker, linking the LytM catalytic domain and the lysostaphin cell wall binding domain for use as disinfectant, antiseptic agent, antibacterial agent and/or anti-inflammatory agent is described.

The term "recombinant" used herein, refers to a protein that is produced in a host cell which is genetically engineered to express the protein. The host can be of viral, mammalian, insect, yeast, bacterial, algal origin. Typically, the host cells are grown in a cell culture by standard procedures and the protein is subsequently obtained from the cell culture by harvesting and, optionally, further purified, by standard techniques such as ion exchange chromatography, affinity chromatography or molecular filtration.

In the present disclosure, the host cell is preferably an *E. coli* cell or a *Pichia pastoris* cell, most preferably an *E. coli* cell.

The term "polypeptide", "peptide" or "protein" refers to a polymer in which most or all of the monomers are amino acids and are joined together by peptide bonds. When the amino acids are α-amino acids, either the L-optical isomer or the D-optical isomer can be used.

Additionally, amino acids not found in nature, e.g., β-alanine, phenylglycine and homoarginine are also included. The amino acids used in the present invention may be the D- or L-isomers, or mixtures thereof.

The term "recombinant polypeptide" according to the invention refers to the chimeric protein containing (i) a LytM catalytic domain, comprising an amino acid sequence shown in SEQ ID NO: 1 or having at least 80% identity thereto which retains the bacteriolytic activity against *Staphylococcus aureus* and wherein the zinc-coordinating amino acids in the active center are not changed and are His in position 26, Asp in position 30, His in position 109 of the SEQ ID NO: 1, (ii) a lysostaphin cell wall binding domain, comprising an amino acid sequence shown in SEQ ID NO: 2 or having at least 80% identity thereto, and (iii) optionally, a peptide linker, linking the LytM catalytic domain and the lysostaphin cell wall binding domain and in the embodiment described as recombinant polypeptide with CPP additionally (iv) from the LssCWT domain side, at the N or C terminus of the polypeptide, has an attached Cell-Penetrating Peptide (CPP) signal sequence directing to the inside of an eukaryotic cell,

The recombinant polypeptides for use of the present invention exhibit bacteriolytic activity against bacteria of the genus *Staphylococcus,* including *S. aureus.*

A LytM catalytic domain refers to a region of the enzyme that interacts with its substrate to cause the enzymatic reaction. LytM is a peptidoglycan hydrolase (autolysin) with glycyl-glycine endopeptidase activity. It acts specifically on polyglycine interpeptide bridges of staphylococcal cell wall peptidoglycan.

Typically, the LytM catalytic domain is defined by the amino acid sequence shown in SEQ ID NO: 1, or having at least 80%, 85%, 90%, 92%, 94%, 95%, 96%, 97%, 98%, or 99% sequence identity thereto.

Preferably, the catalytic domain originates from the LytM enzyme from *S. aureus.*

In the recombinant polypeptides for use according to the invention, the LytM catalytic domain is fused with the lysostaphin cell wall binding domain, optionally via a peptide linker, as disclosed herein. Thereby, the catalytic domain is capable of cleaving the cell wall with the aid of the cell wall binding domain targeting the chimeric enzyme to the cell wall of bacteria of *Staphylococcus,* even more preferably *Staphylococcus aureus.* The pentaglycine cross bridges of *Staphylococcus aureus* peptidoglycan are cleaved by the chimeric enzyme comprising the LytM catalytic domain and the lysostaphin cell wall binding domain.

A lysostaphin cell wall binding domain refers to a motif, which recognizes and binds with the bacterial outer surface. Lysostaphin (EC 3.4.24.75; glycyl-glycine endopeptidase) is a metalloendopeptidase from *S. simulans.*

In Gram-positive bacteria, the outer surface of the bacteria is typically the murein/peptidoglycan layer. Thus, the preferred binding element for these targets will be cell surface entities, whether peptide, protein, lipid, sugar, or a combination thereof. The cell walls of *Staphylococcus* are cleaved, particularly preferably the cell walls of *Staphylococcus aureus* are cleaved. Typically, the lysostaphin cell wall binding domain is defined by the amino acid sequence shown in SEQ ID NO: 2, or having at least 80%, 85%, 90%, 92%, 94%, 95%, 96%, 97%, 98%, or 99% sequence identity thereto.

The cell wall binding domain is fused with the LytM catalytic domain, optionally via a peptide linker, as disclosed herein. Thus, the cell wall binding domain serves to direct the catalytic domain to the cell wall of the bacterium to be eliminated, preferably *Staphylococcus,* particularly preferably *Staphylococcus aureus.*

Optionally, a peptide linker links the LytM catalytic domain and the lysostaphin cell wall binding domain. Optionally means that the present invention works also in the absence of the peptide linker, for example, by direct fusion of the catalytic domain and cell wall binding domain. Typically, the peptide linker comprises from 5 to 35 amino acids, preferably from 10 to 30 amino acids, even more preferably, from 15 to 25 amino acids. In a preferred embodiment, the linker comprises about 17 amino acids.

The peptide linker used in the chimeric polypeptide for use according to the invention may have any amino acid sequence because it is involved neither in the cell wall targeting function of the cell wall binding domain nor in the enzymatic function of the catalytic domain of the chimeric polypeptide, but serves as a linker that most likely provides steric flexibility of the two domains within the polypeptide.

Preferably, the linker comprises the amino acid sequence AGYGKAASTVTPTPNTG (SEQ ID NO: 3) or a sequence having at least 70%, 75%, 80%, 85%, 90%, 92%, 94%, 95%, 96%, 97%, 98%, or 99% sequence identity thereto.

The wild-type linker sequence from lysostaphin (UniProt P10547) comprising the amino acid sequence AGYGKAGGTVTPTPNTG (SEQ ID NO: 4) can also be used as the linker.

The term "diagnostic reagent" refers to a diagnostic reagent detecting staphylococcal presence or absence in a sample, wherein binding of the described recombinant polypeptide to bacteria of the genus *Staphylococcus* is detected via a signal generating moiety attached to the recombinant polypeptide. The signal may also result from lysis of the staphylococcal cells by the described recombinant polypeptide. This might be detected either by changes in optical density of bacterial cultures, release of nucleic acids detected by colorimetric or fluorimetric markers, changes in conductivity, release of particular substances that can be detected in specific assays or any other biochemical, biophysical or optical method.

The diagnostic reagent may be part of a diagnostic kit.

The sample may be blood, saliva, urine, water, food (milk, milk products, meat, fish, sea food etc.), swaps taken from various parts of human or animal body, as well as any surfaces which might be contaminated/colonized by bacteria of the genus *Staphylococcus.*

The term "disinfectant" refers to a composition that destroys vegetative forms of microorganisms, such as bacteria, especially on inanimate objects. The disinfectant as described may comprise the recombinant polypeptides as defined above. Thus, the disinfectant acts by binding to the outer surface of bacteria of the genus *Staphylococcus,* ultimately lysing, and therefore eliminating them. Preferably, the bacteria are *Staphylococcus aureus.* Typically, disinfectants are used on inanimate objects, such as devices and equipment, surfaces of any kind, e.g., of furniture, floors. They are commonly used in hospitals, reception rooms and dental offices, sports facilities, production lines, in food factories and processing plants, as well as in kitchens and bathrooms.

The term "antiseptic agent" refers to a composition that prevents or inhibits the growth of microorganisms, such as bacteria. The antiseptic as described may comprise the recombinant polypeptides as defined above. Thus, such antiseptic agent acts by binding to the outer surface of bacteria of the genus *Staphylococcus,* ultimately lysing, and therefore eliminating them. Preferably, the bacteria are *Staphylococcus aureus.* Typically, antiseptic agents are used on living tissue or skin to reduce the possibility of infection or sepsis.

The term "antibacterial agent" refers to an agent, which is directed against the outer surface of bacteria and therefore, lysing and effectively killing bacteria. The antibacterial agent comprises pharmaceutical or veterinary compositions. The antibacterial agent as described may comprise the recombinant polypeptides as defined above. The antibacterial agent may simultaneously act as an anti-inflammatory agent.

The term "anti-inflammatory agent" refers to an agent counteracting inflammation. The anti-inflammatory agent counteracts inflammation by targeting the cell wall of bacteria and killing the bacteria, thus not eliciting an immune response, and therefore, not provoking inflammation. The are described anti-inflammatory agent comprising the recombinant polypeptides as defined above. The anti-inflammatory agent may simultaneously act as an antibacterial agent.

The recombinant polypeptide for use according to the invention is for use in the prevention and/or treatment of a disorder caused by bacteria of the genus *Staphylococcus,* particularly a dermatological disorder and/or inflammation.

The term "prevention" refers to a reduction in risk of acquiring or developing a disorder caused by bacteria of the genus *Staphylococcus,* particularly a dermatological disorder and/or inflammation, i.e. causing at least one of the clinical symptoms not to occur in a human or non-human animal that may be exposed to bacteria of the genus *Staphylococcus.* Prevention also relates to a reduction in risk of acquiring or developing a disorder caused by bacteria of the genus *Staphylococcus,* particularly a dermatological disorder and/or inflammation, i.e. causing any clinical symptoms not to occur in a human or non-human animal that carries bacteria of the genus *Staphylococcus.* Hence, prevention also refers to the elimination of bacteria of the genus *Staphylococcus* in human or non-human animal carriers that do not experience any clinical symptoms. In other words, limiting or preventing the transmission of bacteria of the genus *Staphylococcus* from one carrier to another is also encompassed by the term "prevention".

The term "treatment" refers to the treatment of a disorder caused by bacteria of the genus *Staphylococcus,* or in which these bacteria are involved, particularly a dermatological disorder and/or inflammation, in particular, the treatment relates to ameliorating the disease and/or condition (i.e., arresting the development of the disease or reducing the extent or severity of at least one of the clinical symptoms thereof). Treatment can also refer to ameliorating at least one physical parameter, which may not be discernible by the human or non-human animal. Treatment may also refer to changes in the symptoms of the disorder: physical, (e.g., stabilization of a discernible symptoms), physiological, (e.g., stabilization of a physical parameter), or both. Alternatively, "treatment" can also refer to slowing the progression of the disorder.

Dermatological disorders are disorders which affect the skin, which is made of layers of ectodermal tissue. The skin is in contact with the environment, and it protects the underlying structures (e.g., muscles, bones, ligaments and internal organs) from contact with the outside environment or from injury, including protection against pathogens such as bacteria. The skin may be affected by disorders that are diverse in terms of cause, presentation and/or treatment. Preferably, , the dermatological disorders are acne, pimples, dermatitis, atopic dermatitis, infections and/or injuries of a mucous membrane or of the skin, wounds, diabetic foot, ulcers, burns and inflammation of the mammary gland, udder, eyes, ears, throat, sinuses or genitals. The mucous membrane is to be understood as the thin skin that produces mucus and covers the inside surface of a part of the body. The mucous membrane comprises one or more epithelial layers such as within the teat/nipple, lactiferous ducts, lactiferous sinus, eye, ear, nasal or respiratory passage and genitals. Diabetic foot is a disease linked with type II diabetes and is associated with a high risk for skin lesions and wounds on the skin of the feet which often develop into chronic wounds. Ulcers are a discontinuity of the skin or a break in the skin or mucous membranes. Ulcers comprise diabetic foot ulcers, a complication of diabetic foot, pressure ulcers, genital ulcers, ulcerative dermatitis and anal fissures. Inflammation is a causative agent of ulcers. The dermatological disorder can occur in isolation or in combination with inflammation as described below. Hence, the dermatological disorder may be associated with inflammation.

Inflammations are disorders which affect the organs by inflammation, i.e. a local response to cellular injury that is manifested by any one or a combination of the following: capillary dilatation, leukocytic infiltration, redness, heat, and pain. Inflammation serves as a mechanism initiating the elimination of noxious agents and of damaged tissue. Thus, the organs may be affected by inflammations that are diverse in terms of cause, symptoms and/or treatment. Inflammation can occur separately or in combination with a dermatological disorder, as described above. Hence, inflammations can also comprise a dermatological component.

The dermatological disorders and/or inflammations which involve the presence of Gram-positive bacteria, particularly bacteria of the genus *Staphylococcus,* particularly *Staphylococcus aureus.* Gram-positive bacteria, particularly bacteria of the genus *Staphylococcus,* particularly of the species *Staphylococcus aureus* may contribute to the development or progression of dermatological disorders. Gram-positive bacteria, preferably bacterium of the genus *Staphylococcus* or more preferably of the species *Staphylococcus aureus* may contribute to the development or progression of an inflammation. The term "Gram-positive bacteria" refers to bacteria in which the outer surface is typically the murein layer. *Staphylococcus* is a genus of Gram-positive bacteria, and can cause a wide variety of diseases in humans and animals through either toxin production or penetration. Staphylococci-related infection can range from mild and requiring no treatment to severe and potentially fatal. Over 30 different species of *Staphylococcus* can infect humans and non-human animals. Manifestations of staphylococcal infections usually depend on the type of infection, and the organism which causes it. Common types of infections include the following: skin infections (e.g., folliculitis, furuncles, impetigo, wound infections, scalded skin syndrome), soft-tissue infections (e.g., pyomyositis, septic bursitis, septic arthritis), mastitis, toxic shock syndrome, purpura fulminans, endocarditis, osteomyelitis, pneumonia, infections related to prosthetic devices (e.g., prosthetic joints and heart valves, vascular shunts, grafts, catheters), and urinary tract infection. Individuals with suppressed immune systems (taking immune-suppressing medications or with immune deficiencies) are at increased risk of developing more serious infections. *Staphylococcus* species comprise: *Staphylococcus arlettae, Staphylococcus aureus, Staphylococcus aureus* NCTC 8325-4, *Staphylococcus auricularis, Staphylococcus carnosus, Staphylococcus carprae, Staphylococcus chromogenes, Staphylococcus cohnii, Staphylococcus delphini, Staphylococcus dysgalactiae, Staphylococcus epidermidis, Staphylococcus equorum, Staphylococcus gallinarum, Staphylococcus hemolyticus, Staphylococcus hominis, Staphylococcus hyicus, Staphylococcus intermedius, Staphylococcus kloosii, Staphylococcus lentus, Staphylococcus lugdunensis, Staphylococcus muscae, Staphylococcus pasteuri, Staphylococcus pettenkoferi, Staphylococcus saprophyticus, Staphylococcus sciuri, Staphylococcus simulans, Staphylococcus warnerii, Staphylococcus xylosus.* Preferably, the *Staphylococcus* species is *Staphylococcus aureus.*

In some of the presented embodiments, the recombinant polypeptide exhibits bacteriolytic activity in raw milk and/or serum. The term "bacteriolytic" is typically used to mean "bacterial cell wall degrading", i.e. degrading the outer surface of the bacteria. Bacteriolytic activity leads to degradation, broking down, disintegration of the cell wall, or diminishing its integrity. Bacteriolytic activity may be an extreme form of cell-wall-degrading activity or the result of such an activity.

Preferably, the raw milk is raw cow's milk and the serum is preferably bovine or human serum. Raw cow's milk is non-processed and undiluted and comprises next to water, fat, lactose, various milk proteins also bacteria of the genus *Lactococci, Lactobacilli* and *Leuconostoc.* If a cow is a carrier of bacteria of the genus *Staphylococcus* or suffers from any form of mastitis, the milk also comprises bacteria of the genus *Staphylococcus* (staphylococci). The pH of cow's milk depends on various factors, i.a., the storage time and the presence of bacteria, including bacteria of the genus *Staphylococcus.* Regular cow's milk has a pH from about 6.0 to 7.0, cow's milk which comprises *Staphylococcus* has increased pH values compared to regular cow's milk. In that case, the pH values may range from 7.0 to 8.5. Hence, raw cow's milk is an emulsion with a highly specific environment, shaped by various factors including the presence of bacteria. Therefore, enzymes that exhibit activity in raw cow's milk need to be specifically targeted to these specific requirements. Similarly, fetal bovine serum stems from the blood of a bovine fetus and therefore, contains a complex array of protein components, in particular growth factors, hormones and amino acids. It also contains carbohydrates and lipids. Bovine serum albumin is the major component of fetal bovine serum. Therefore, enzymes that exhibit activity in raw cow's milk need to be specifically target to these specific requirements. Human serum corresponds to blood plasma without any coagulation factors. It comprises a complex array of protein components, in particular, growth factors, hormones and amino acids. It also contains carbohydrates and lipids.

The recombinant polypeptide may be formulated for topical administration. Topical refers to a mode of administration, and means that a material is administered by being applied to the skin or to the mucous membrane, which comprises one or more epithelial layers such as within the nipple, lactiferous canal, lactiferous sinus, eye, ear, nose or respiratory passage and genitals. The recombinant polypeptide for topical administration may be applied locally by external lubrication of the skin in a thin layer for prevention and/or treatment of mammary gland and/or udder tissue inflammations (including the teat/nipple, lactiferous canal, lactiferous ducts, and nipples), in particular for the treatment and/or prevention of mastitis. The recombinant polypeptide for topical administration may be applied locally by rinsing or dipping the affected area. Alternatively, the topical administration form can be applied by dropping it on or into the affected area. Preferably, the formulation is in the form of a cream, suspension, lotion, solution, ointment, gel, foam, transdermal patch, powder, paste, tape, pad, swab, wound dressing or compress.

In some embodiments, the recombinant polypeptide is formulated for intramammary delivery. The intramammary delivery refers to a form of delivery, in which the recombinant polypeptide is delivered via lactiferous ducts into the udder/teat/breast by injection, infusion or rinsing.

In some examples, the embodiment is referred to a recombinant polypeptide which is delivered to a teat or nipple, preferably by infusion, rinsing or dipping. The teat encompasses the nipple/teat, lactiferous ducts and lactiferous sinus. Infusion refers to the delivery of a composition comprising the recombinant polypeptide directly into the body site, where the composition comprising the recombinant polypeptide is required. Rinsing refers to cleansing by flushing with a composition comprising the recombinant polypeptide, thereby removing potential bacteria and simultaneously, specifically damaging the potential remainders of bacteria.

It is described the delivery of a recombinant polypeptide to a female human or non-human animal suffering from mastitis or at risk of developing mastitis, preferably a ruminant, more preferably a dairy cow. A female shall be understood as an individual harboring mammary glands and can therefore, lactate. The non-human animal according to the present description is a mammal. Ruminants comprise cattle, domesticated and wild bovines, goats, sheep, giraffes, deer, gazelles, and antelopes. Dairy cows comprise female cows which have the ability to lactate. At risk of developing mastitis refers to the risk of acquiring or developing the mastitis, i.e. causing at least one of the clinical symptoms to occur in a subject that may be exposed to, i.e. lactiferous canals/ducts and/or teat/nipple inflammation, in particular to mastitis, or predisposed to lactiferous canals/ducts and/or teat/nipple inflammation, in particular to the mastitis in advance of its onset. Alternatively, at risk of developing mastitis may also refer to the risk of acquiring or developing the mastitis, i.e. causing none of the clinical symptoms to occur in a subject that may be exposed to, if the subject is a carrier of bacteria of the genus *Staphylococcus.* Suffering from mastitis refers to the persistent, inflammatory reaction of the mammary gland and udder tissue, and is a major endemic disease of dairy cattle. It usually occurs when leukocytes are released into the mammary gland, as an immune response to bacterial invasion of the teat canal by variety of bacterial sources. Mastitis, in particular dairy animal mastitis, can be caused by many different bacterial species, one of the most common of which are staphylococci. Milk-secreting tissue and various ducts throughout the mammary glands are damaged due to toxins released by the bacteria.

Another described aspect relates to administration of a veterinary composition for use.. The veterinary composition for use comprising the recombinant polypeptide according to the invention may also comprise at least one excipient which modifies the release of the recombinant polypeptide and/or modifies the biodegradation of the recombinant polypeptide and/or stabilizes the at least one recombinant polypeptide during manufacturing of, storage and release. Moreover, the excipient may modify the solubility of the recombinant polypeptide. The excipient can, e.g., be a hydrophilic polymer, a sugar, a polyol, a surfactant, an antioxidant and/or a water-soluble salt or any other excipient known to achieve the abovementioned purposes.

Moreover, further excipients may be added to the veterinary composition for use comprising the polypeptide in order to optimize its chemical, physical and mechanical properties, in order to adapt it for the intended use. These additives include inter alia sodium benzoate, fatty acid esters or salts, trisodium phosphate, liquid paraffin, zinc oxide, calcium stearate, zinc stearate. The recombinant polypeptide formulated for topical administration may also contain other excipients, e.g. in order to improve the technical properties of the veterinary composition for use comprising the recombinant polypeptide, so that it may be easier to produce or in order to improve the stability. A suitable excipient approved for pharmaceutical use, for use in a veterinary composition for use according to the invention may be selected from the group consisting of fillers, diluents, disintegrants, glidants, pH-adjusting agents, viscosity adjusting agents, solubility increasing or decreasing agents, osmotically active agents and solvents.

The veterinary composition for use may further comprise excipients approved for pharmaceutical use, in order to increase the chemical stability. Preferred excipients are, e.g., chelating agents such as EDTA, disodium EDTA, deferoxamine, lipoic acid, glutathione, or antioxidants such as ascorbic acid palmitate, alpha-tocopherol, ubiquinol, β-carotene, retinol and hydrophilic and/or lipophilic antioxidants.

It is described within specification the use of the veterinary composition for use in the prevention/and or treatment of dermatological disorders and/or inflammations. caused by a bacterium of the genus of *Staphylococcus.* Dermatological disorders are disorders which affect the skin, which is made of layers of ectodermal tissue. The skin protects the underlying structures (e.g., muscles, bones, ligaments and internal organs) from contact with the outside environment or from injuries, including protection against pathogens such as bacteria. The skin may be affected by disorders that are diverse in terms of cause, presentation and/or treatment. The dermatological disorder may also be associated with inflammation. Inflammations are disorders which affect different organs by inflammation, i.e. a local response to cellular injury that is marked by any one or a combination of the following symptoms: capillary dilatation, leukocytic infiltration, redness, heat, and pain. Inflammation serves as a mechanism initiating the elimination of noxious agents and of damaged tissue. Thus, the organs may be affected by disorders that are diverse in terms of cause, symptoms and/or treatment. Inflammations can occur separately or in combination with a dermatological disorder, as described above. Hence, inflammations can also comprise a dermatological component.

It is described the veterinary composition for use, wherein the dermatological disorder and/or inflammation caused by a bacterium of the genus of *Staphylococcus* is selected from the group consisting of dermatitis, injuries and/or infections of a mucous membrane or of the skin, wounds, ulcers, and an inflammation of the udder, eye, ear, throat, sinuses or genitals. The mucous membrane is the thin membrane that produces mucus and covers the inside surface of parts of the body. The mucous membrane comprises one or more epithelial layers such as within the teat, teat canal, teat cistern, eye, ear, nasal or respiratory passage and genitals. Ulcers are a discontinuity of the skin or a break in the skin or mucous membranes. Ulcers comprise pressure ulcers, genital ulcers, ulcerative dermatitis and anal fissures. Inflammation is a causative agent of ulcers.

It is described, the veterinary composition for comprising the recombinant polypeptide is formulated for intramammary administration. This administration refers to a form of delivery, wherein the recombinant polypeptide is delivered to a nipple or teat, by, for example, infusion, rinsing or dipping. The teat encompasses the teat/nipple, teat canal and teat cistern. Infusion refers to the delivery of a composition comprising the recombinant polypeptide directly into the body site, where the composition comprising the recombinant polypeptide is required. Rinsing refers to cleansing by flushing with a composition comprising the recombinant polypeptide, thereby removing potential bacteria and simultaneously, also the remainders of bacteria.

It is described the veterinary composition for use is provided for use in the treatment of mastitis in ruminants. Ruminants comprise cattle, domesticated and wild bovines, goats, sheep, giraffes, deer, gazelles, and antelopes. Dairy cows comprise female cows which have the ability to lactate. Suffering from mastitis refers to the persistent, inflammatory reaction of the mammary gland and udder tissue, and is a major endemic disease of dairy cattle. It usually occurs when leukocytes are released into the mammary gland, as an immune response to bacterial invasion of the teat canal by different bacteria. Mastitis, in particular in dairy cows, can be caused by many different bacterial species, the most common among them being *Staphylococcus.* Milk-secreting tissue and various ducts throughout the mammary glands are damaged due to toxins released by the bacteria.

The concentration of the recombinant polypeptide, when used as a disinfectant, will typically be at least 10 nM, preferably at least 20 nM, more preferably at least 50 mM, and most preferably at least 100 nM. Typically, the concentration will be in the range from 10 nM to 1000 nM, preferably in the range from 20 nM to 500 nM, more preferably in the range from 20 nM to 200 nM. However, the effective concentration will depend on the formulation, the conditions applied, the environment etc., and can be even higher than 1 mM.

The concentration of the recombinant polypeptide, when used as an antibacterial agent within a pharmaceutical or veterinary composition for use , will typically be at least 10 nM, preferably at least 50 nM and more preferably at least 100 mM. Typically, the concentration will be in the range from 10 nM to 1000 nM, preferably in the range from 50 nM to 1000 nM, more preferably in the range from 100 nM to 1000 nM. However, the effective concentration will depend on the formulation and the conditions applied and can be even higher than 1 mM. This also applies to the use in intramammary administration in mastitis.

The recombinant polypeptide for use according to the invention can be used in combination with another active agent, such as an antibiotic or antiseptic agent. Examples of antibiotics that can be used in a combination therapy with the polypeptide for use according to the invention are vancomycin, erythromycin, daptomycin, and ciprofloxacin. Examples of antiseptics that can be used in combination with the recombinant polypeptide for use according to the invention are chlorhexidine, iodine, silver nanoparticles, and oxygen peroxide (up to 0.03%).

The recombinant polypeptide for use according to the invention can also be advantageously used in combination with detergents such as SDS (preferably up to 0.01%), polysorbate 20 (Tween 20; preferably up to 0.01%), polysorbate 80 (Tween 80; preferably up to 0.1%), octoxynol-9 (Triton X-100; preferably up to 0.1%), sorbitane monooleate (Span 80; preferably up to 0.1%), and common detergents as in dishwashing liquids and softeners.

The invention also relates to a cosmetic or care composition for external use, and thus improvement of the condition and appearance of the skin, especially in the case of skin infected with staphylococci, as well as acne skin by limiting the amount of staphylococci bacteria on the skin or eliminating their occurrence. The skin is subjected to various processes of destruction by dermatological diseases and undesirable cosmetic conditions, especially staphylococci and acne infections, which are often co-infected or secondarily infected with staphylococci. We have unexpectedly found further undisclosed use of the new recombinant polypeptide according to the invention in the form of a cosmetic or care composition for cosmetic, care and hygiene use in humans and animals, for application on the skin to provide previously undisclosed treatment, preventive and care benefits. We have found that it is possible to effectively treat and prevent dermatological conditions and undesirable cosmetic conditions caused by staphylococci, particularly in acne co-infected with staphylococcus.

The cosmetic or care composition for use according to the invention also contains a dermatologically/cosmetically acceptable carrier that acts as a diluent, dispersant or carrier for the active ingredients. The carrier may include materials commonly used in skin care products such as water, liquid or solid emollients, silicone oils, emulsifiers, solvents, humectants, thickeners, powders, propellants and the like. In addition to the active substance, which is the recombinant peptide, other specific active substances beneficial to the skin, such as agents preventing the harmful effects of sunlight, agents with care and antibacterial properties, e.g., plant extracts or zinc, silver or copper ions, may also be included in the cosmetic or care composition for use. The carrier may also contain adjuvants such as fragrances, opacifiers, preservatives, dyes and buffers. Typical methods for making skin care products can be used to produce the composition for external use. Oil-in-water or water-in-oil emulsions are preferred compositions. The compositions may be in the form of conventional care products such as cream, gel or lotion, ointment, lotion, powder or the like. It was shown that these active substances, carriers and additives used in the described compositions are inert to the enzymatic activity of the recombinant peptide, and can be preferably used to support and/or enhance the enzymatic activity of the recombinant peptide in the various applications mentioned herein.

### EXAMPLES

In the following examples, unless it was otherwise indicated, standard materials and methods used in the field were used, or the manufacturers' instructions for specific materials and methods were followed.

### Example 1: Cloning and expression of chimeric enzymes

This example includes the preparation and delivery of two chimeric enzymes A and B that comprise the LytM catalytic domain, the lysostaphin cell wall binding domain of and a peptide linker.

### Cloning

The catalytic domain (LytMCD, residues 185-316), the peptide linker (Lss_linker) and the lysostaphin cell wall binding domain (LssCWT) were ligated using overlap extension PCR in two different arrangements **(****Fig. 1****).** The primer is constructed so that it has a 5' overhang, which is complementary to the end of another DNA molecule. After annealing and extension of the DNA, the obtained DNA fragments are multiplied by PCR using another pair of primes which are complementary to the target DNA ends. The obtained PCR products were purified and digested with NcoI and XhoI enzymes and ligated with similarly digested pET15b expression vector. *E. coli* TOP10 strain was transformed and after sequence verification, plasmids were transformed into *E. coli* BL21 (DE3) strain. Overexpression of proteins was induced by 1 mM IPTG and performed for about 4 hours at 25 °C.

### Mutagenesis

Double mutants of the linker in Chimera A and Chimera B were produced by PCR-based site-directed mutagenesis with Phusion high-fidelity DNA polymerase (Thermo Fisher Scientific, Waltham, USA) using pET15b-ChimeraA/ B/ plasmids as a PCR template. The presence of the introduced mutations was confirmed by DNA sequencing.

### Attachment of signal/targeting sequences

Constructs were produced, in which sequences that were signal/targeting sequences to the inside of an eukaryotic cell, were attached at the C-terminus of Chimera A and N-terminus of Chimera B. Signal sequences targeting the protein into the inside of a eukaryotic cell (Cell-Penetrating Peptides, CPPs) were cloned at the C-terminus of Chimera A into the XhoI restriction site or at the N-terminus of Chimera B into the NcoI restriction site. Attachment of the CCP sequences enables easy passage through the cell membrane and transport into the cell, e.g., into cytoplasm or cell organelles.

DNA sequences of CPPs in the form of single-stranded oligonucleotides, designed so that the ends are recognized by the restriction enzyme, were hybridized, and then ligated with linearized vectors containing Chimera A or B sequences. The ligation mixture was transformed into *E. coli* TOP10 competent cells, then positive clones were sequenced, and *E. coli* BL21 (DE3) competent cells were transformed with the vectors with confirmed sequence. Protein overproduction was carried out in an auto-induction medium overnight at 25 °C. Expression levels were verified on a polyacrylamide gel.

The sequences of the attached CPP signal sequences for which constructs with Chimera A and B were produced are given below in **Table 2.** It should be noted that other CPPs are known that can also be attached to the recombinant peptide, to direct it into the cell.

**Table 2**

| **SEQ ID No** | **Amino acid sequence** | **CPP name** |
|---|---|---|
| 9 | RQIKIWFQNRRMKWKK | Penatrin |
| 10 | WEAKLAKALAKALAKHLAKALAKALKACEA | CPP 2 |
| 11 | MVTVLFRRLRIRRASGPPRVRV | CPP 3 |
| 12 | KLALKLALKALKAALKLA | CPP 4 |
| 13 | RRQRRTSKLMKR | CPP 5 |
| 14 | LLIILRRRIRKQAHAHSK | CPP 6 |
| 15 | RRRRRRRR | poly-arginine |
| 16 | GRKKRRQRRRPPQ | HIV-TAT |
| 17 | GWTLNSAGYLLGKINLKALAALAKKIL | CPP 9 |
| 18 | AGYLLGKINLKALAALAKKIL | CPP 10 |
| 19 | RKKRRQRRR | CPP 11 |

The schematic structure of Chimera A with attached CPP, and the sequences of the recombinant peptides produced with the CPP sequences attached at the C-terminus are shown in **Fig. 1D****.** A schematic structure of Chimera B with CPP attached at the N-terminus is shown in **Fig. 1E**. The sequences of the produced recombinant peptides, with CPP sequences attached at the N-terminus of Chimera B had the CPP sequences shown in **Table 2.**

### Protein purification

The pellet from *E. coli* cultures containing the overexpressed proteins was resuspended in 20 mM of Tris-HCl pH 7.0, 1 M NaCl, 10% glycerol and sonicated. Cell lysate was dialyzed in 20 mM Tris-HCl, pH 7.0, 50 mM NaCl and purified by ion exchange chromatography using a NaCl gradient (SP Sepharose column, GE Healthcare). Further purification was performed by gel filtration on the Superdex 75 column (GE Healthcare, Uppsala, Sweden) in the buffer: 20 mM Tris-HCl pH 7.0, 200 mM NaCl, 10% glycerol.

Anti-staphylococcal activity of the purified chimeric enzymes was monitored by assays of turbidity reduction in bacterial cultures. Reference strains of *S. aureus* (NCTC 8325-4) were grown in Tryptic Soy Broth (TSB) medium at 37 °C. The turbidity assays were performed in 96-well microtiter plates. Bacterial cells used in all experiments were collected in their exponential growth phase, suspended in TSB, washed, and resuspended in an appropriate buffer to obtain OD₅₉₅ of 1.0 (corresponding to 10⁸ CFU/ml). Cell lysis was monitored for 1 hour at room temperature. Readings were performed every 10 minutes after 5 seconds of shaking of the sample. Tests were carried out in 50 mM glycine buffer, pH 8.0, 100mM NaCl. The lytic activity of both Chimeras (A and B) was nearly identical **(****Fig. 2****).** For this reason, further described in detail are only the experiments performed using Chimera A.

The produced Chimeras A and B with attached CPP signal sequences were characterized by a similar activity.

### Example 2: Chimera A is active against Staphylococcus causing mastitis

The aim of this example is the isolation of bacterial strains from milk of dairy cows suffering from mastitis and testing their susceptibility to an enzyme- in this case Chimera A. Milk samples were taken from cows whose somatic cell counts (SSC) were increased. The increased SCC is indicative of mastitis. Milk samples were taken from each quarter of the udder separately and plated on CHROMagar Mastitis (GrasoBiotech, Owidz, Poland). After 18 hours incubation at 37 °C, single colonies of different morphologies were plated on fresh medium and grown for another 18 hours at 37 °C. Isolated colonies were identified using the VITEK System (bioMerieux).

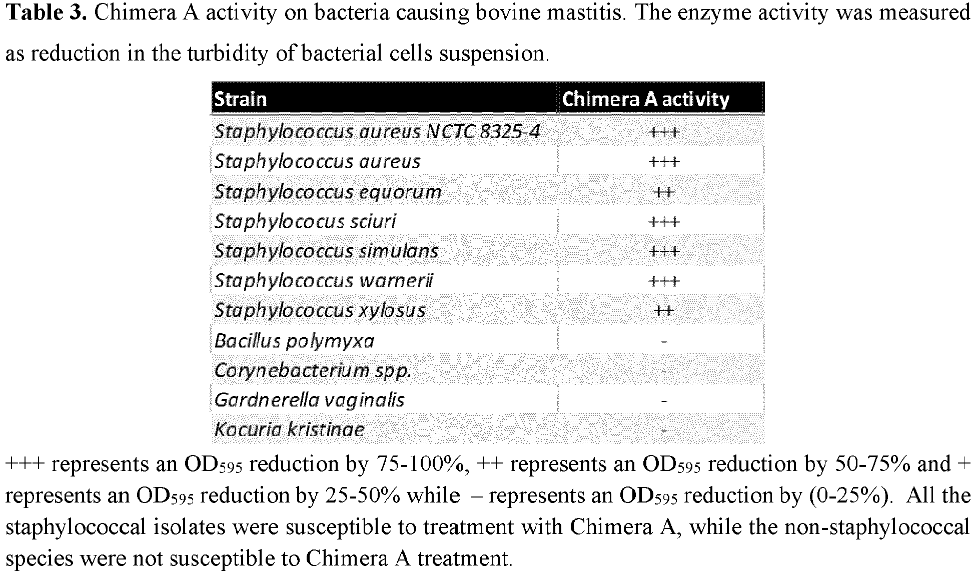

### Example 3: Chimera A is active in UHT milk

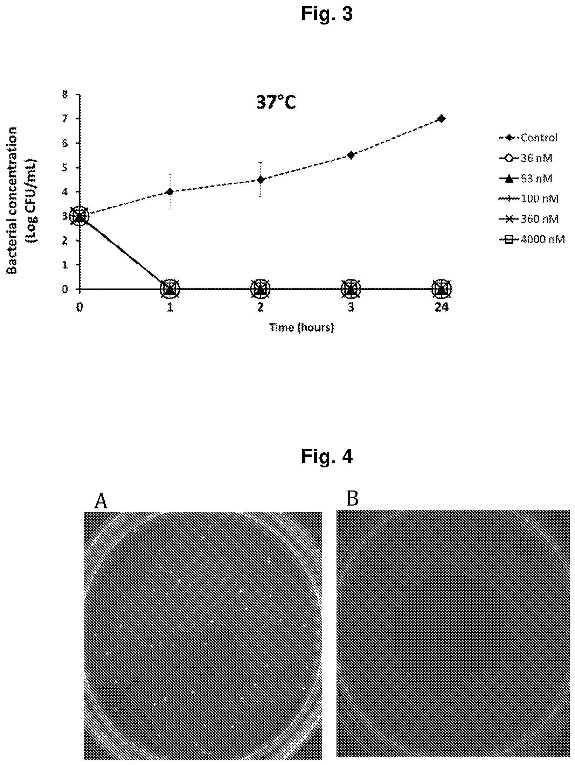

The aim of this example is to demonstrate the activity of the chimeric enzyme in UHT milk. The activity of Chimera A in UHT milk was estimated based on the reduction in the number of colonies (CFU) of *Staphylococcus aureus* in 1 ml after incubation in milk, in the presence of the tested enzymes. *Staphylococcus aureus* NCTC 8325-4 strain was grown to OD₅₉₅ of 0.6 in TSB at 37 °C with mild agitation. The activity of Chimera A at 37 °C was determined for the initial number of cells at a concentration of 10³ CFU/ml and for various enzyme concentrations (36 - 4000 nM). Even at concentrations of Chimera A as low as 36 nM, *S. aureus* was completely eradicated from milk to an undetectable level within 1 hour of incubation **(****Fig. 3** and **Fig. 4****).**

UHT milk was inoculated with *S. aureus* in the amount of 10⁶ CFU/ml and incubated at 4 °C or room temperature in the presence of 100 nM of the enzyme. Samples were taken after 2 and 24 hours of incubation and the bacterial count was determined by serial dilution and plating on TSB-agar plates. Plates were incubated at 37 °C for 18 hours. Milk incubated without enzymes was used as a negative control. Experiments were performed in three independent repeats.

Chimera A eliminated *S. aureus* from UHT milk, both at 4 °C and 22 °C **(****Fig. 5****),** and was much more effective at room temperature (reduction of CFU/ml by 8 orders of magnitude). The initial number of staphylococcal cells (1 million/ml) was reduced by at least 99.9% in just 2 hours of incubation with the enzyme.

### Example 4: Chimera A is active in raw cow's milk

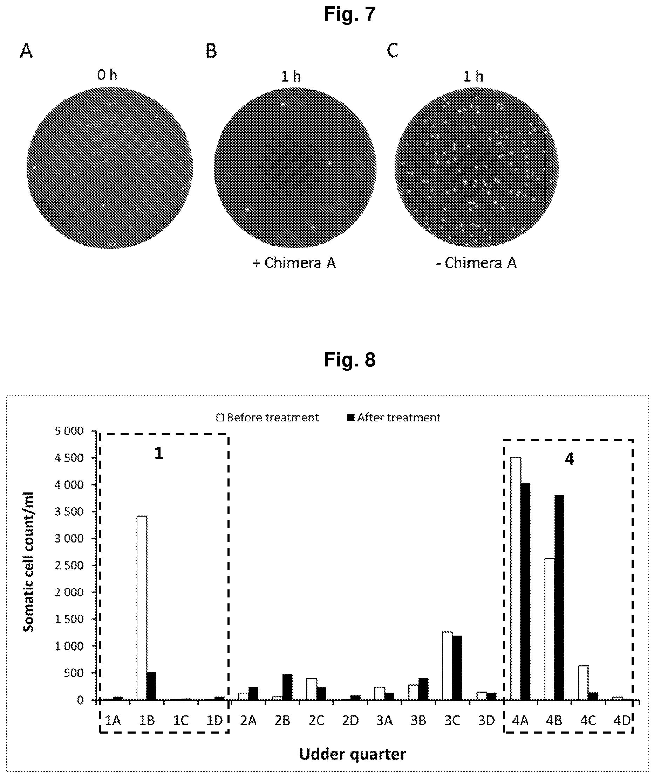

The aim of this example is to demonstrate the activity of the chimeric enzyme in raw cow's milk. In order to determine the effect of raw milk on the activity of the chimeric enzyme, raw cow's milk with the addition of *S. aureus* was incubated in the presence of 36 and 100 nM of Chimera A for 3 hours. The activity of Chimera A in raw cow's milk was determined as a decrease in CFU/ml log over time. Raw milk was inoculated with the NCTC 8325-4 *S. aureus* strain, cultured to an OD₅₉₅ of 0.6 in TSB at 37 °C with mild agitation, at a concentration of 10³ CFU/ml. The inoculated milk was incubated in the presence of various concentrations of Chimera A at 37 °C. Samples were taken after 0, 1, 2 and 3 hours, plated on TSB-agar plates, which were incubated at 37 °C for 18 hours. The surviving cells were counted and CFU/ml log decrease was estimated. The sample incubated without the enzyme served as negative control. Experiments were repeated three times. Chimera A eliminated over 99% of the initial cells number within 1 hour **(****Fig. 6A** and **Fig. 7****).** In subsequent tests, Chimeras' ability to eliminate greater number bacteria was checked. 10⁶ CFU/ml of *S. aureus* (strain NCTC 8325-4) cells, present in milk were treated with 100 nM of Chimera A at 22 °C for 24 hours. After just two hours, the number of bacterial cells decreased to 10³ CFU/ml and after 24 hours, 100% of the bacterial cells were eliminated **(****Fig. 6B****).**

### Example 5: Chimera A causes a decrease in somatic cell counts in milk obtained from mastitis-affected cows

The aim of this example is to demonstrate that somatic cell counts decreased in milk samples from mastitis-affected cows after treatment with Chimera A. Four cows belonging to an experimental herd of dairy cattle stemming from an experimental farm were selected for the experiment. The selection of the cows was based on the somatic cell count over the past 8 months; the selected cows had an increased somatic cell count in at least one quarter of their udder, which indicated different stages of mastitis development. Immediately prior to the start of the experiment, the somatic cell counts were determined in milk collected from each quarter and the obtained milk sample underwent microbiological analysis. Bacteria were cultured and the species were identified as described in Example 1. The same analysis was performed for milk collected at the end of the experiment.

The enzyme solution contained 20 mM of Tris-HCl at pH 7.4, 100mM NaCl, 10% glycerol and 100 nM of Chimera A. Immediately after milking, each quarter was washed with the enzyme solution. This treatment was repeated twice a day, for seven days.

In the case of cow 1, the initial somatic cell count was almost 3.5 million in the milk obtained from quarter B, which was representative for acute mastitis. In the other quarters (A, C, D), the somatic cell count did not exceed 20,000 which indicates lack of inflammation **(****Fig.8****).** Microbiological tests of milk samples demonstrated that the somatic cell count and therefore, inflammation of quarter B was caused by *Staphylococcus.* After seven days of udder treatment with Chimera A solution, the somatic cell count of the milk obtained from quarter B was reduced highly significant to slightly above 500,000. The somatic cell count of the other quarter (A, C, D) changed slightly and did not exceed 100,000, which is a standard level for healthy animals and indicates the absence of infection. The staphylococcal species isolated from the infected quarter were identified as *S. xylosus.* In the *in vitro* Chimera A susceptibility tests, this staphylococcal strain proved to be sensitive to the used chimeric enzyme **(Table 3).**

A similar effect was observed in cow 4 whose somatic cell counts indicated acute inflammation in two teats (udder quarters): teat A and B (somatic cell count above 4.5 million and 2.6 million, respectively) and a slightly less severe mastitis in teat C (SCC) over 600,000. The fourth quarter (D) did not show any signs of inflammation (somatic cell count of 54,000). The microbiological analysis of the milk samples revealed the presence of *Staphylococcus* only in teat C. Solely the associated quarter of teat C showed a significant reduction of the somatic cell count from above 623,000 to 150,000 after treatment with the chimeric enzyme solution for one week. In all other quarters, no significant changes in somatic cell count were observed, also no pathogenic staphylococcal species were detected in milk samples taken from these quarters.

### Example 6: Chimera A is active in serum

The aim of this example is to demonstrate the activity of the chimeric enzyme in serum. The lytic activity of 100 nM solution of the Chimera A enzyme was tested in turbidity reduction assays in the presence of fetal bovine serum. The test was performed in 96-well microtiter plates.

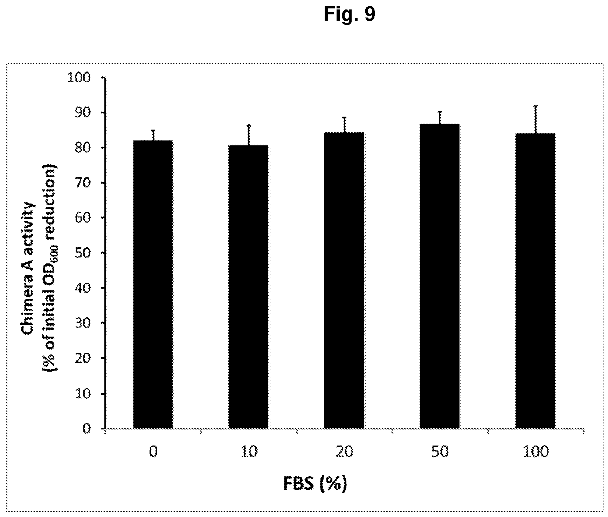

*Staphylococcus aureus* NCTC 8325-4 reference strain was cultured to logarithmic growth phase, washed and suspended in 50 mM glycine buffer, pH 8.0, 100 mM NaCl to obtain OD₅₉₅ of 1.0. Tests were carried out in 50 mM glycine buffer, pH 8.0, 100mM NaCl with the addition of fetal bovine serum (FBS) at the final concentration from 0 to 100%. The decrease in OD₅₉₅ was monitored for 1 hour at room temperature, with readings every 10 minutes, after 5 seconds of shaking. The positive control did not contain FBS. The activity of the chimeric enzyme was not altered in the presence of FBS, even at the concentration of 100% **(****Fig. 9****).**

In order to confirm that the chimeric enzyme is as active in 100% serum as in optimal conditions, the number of live cells was determined and showed that the enzyme eliminated more than 99.99% (5 orders of magnitude decrease) of staphylococcal cells within 2 hours **(Table 4).** The same result was obtained for the activity of the chimeric enzyme in glycine buffer.

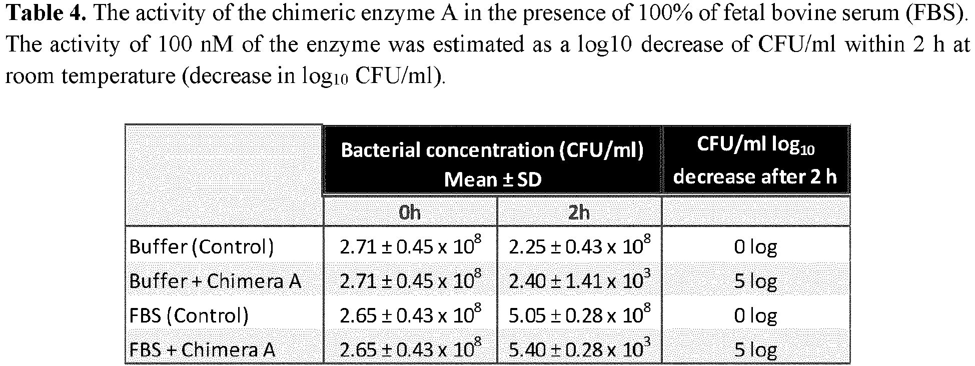

### Example 7. Comparative examples

### A. Comparison of chimeras' activity

Verbree et al. have published the results of analyzes of about 170 different lysins of phage origin and various modified chimeras tested for activity in milk (Verbree, Datwyler et al., 2018). Only 8 of the 170 enzymes tested showed limited activity in pasteurized milk, but none of them was effective in removing staphylococcus cells from raw milk. A slight decrease in the number of staphylococcus cells in raw milk was observed only in mixtures of different bacteriolytic enzymes administered simultaneously and at very high concentration. None of the tested enzymes was effective in eliminating large numbers of bacterial cells at low enzyme concentrations.

We compared the activities of the chimeras - Chimera A - by conducting experiments with them under identical conditions and concentrations in which the chimeras described in Verbree, Datwyler et al. (2018) were tested. **Table 5** shows the obtained comparative results presented as orders of magnitude of the number of cells, and orders of decrease in the number of cells (Δ) after treatment with appropriate enzymes.

**Table 5. Comparison of the effects of the enzymes with the results of tests of other chimeras presented in Verbree, Datwyler et al., 2018.**

| Test conditions | | | | Cell count after three hours of treatment with the enzyme log₁₀ | | | |
|---|---|---|---|---|---|---|---|
| Milk | Enzyme concentr ation | Control cell count log₁₀ | | Results Verbree et al. | | Results Chimera A | |
| | | T₀ | T3c | T_{3E} | **Δ** | T_{3E} | **Δ** |
| UHT | 36 nM | 3 | 5 | 4-5 | **0-1** | 0 | **5** |
| UHT | 360 nM | 3 | 5 | 0-4 | **1-5** | 0 | **5** |
| UHT | 4000 nM | 3 | 5 | 3-0 | **2-5** | 0 | **5** |
| RAW 100% | 36 nM | 3 | 4 | - | - | **0** | **4** |
| RAW 100% | 320 nM | 3 | 4 | 4-1 | **1-3** | - | |

| | | | | | | | |
|---|---|---|---|---|---|---|---|
| Number of bacteria in orders of magnitude. T₀ - initial; T_{3C} - after 3 hours without the enzyme, T_{3E} - after 3 hours with the enzyme, Δ - decrease in the number of bacteria after 3 hours of treatment with the enzyme, expressed in orders of magnitude. RAW- raw milk. | | | | | | | |

It was shown that Chimeras according to the invention exhibit many times higher activity, even when used at very low concentrations. The high activity of the Chimeras according to the invention was observed not only in UHT milk; it was also found that these Chimeras are very effective in removing staphylococcus cells from raw milk, a property not shared by any chimera known in the state of the art.

### B. No activity of a chimera known in the state of the art.

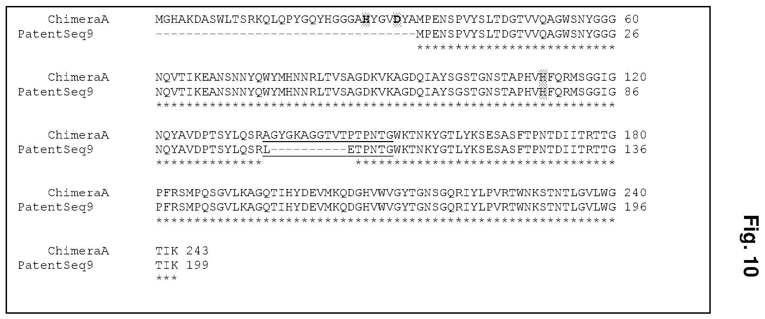

In the patent application WO2007/130655 from Ganagagen, chimeric enzymes consisting of the active LytMCD domain and the lysostaphin cell wall binding domain were described. Alignment of the amino acid sequence of Chimera A according to the invention to the chimera shown in Seq Id No 9 in the WO2007/130655 publication is shown in **Fig. 10****.**

As can be seen from the presented alignment, the chimera sequence Seq ID No 9 known in the state of the art includes only a part of the peptide building catalytic domain, and it lacks 34 amino acids at the N-terminus, including two coordinating zinc residues H210 and D214, which have been experimentally shown to be necessary for the operation of the LytM catalytic domain (Odintsov, Sabala et al., 2004) and other members of this family, such as Ale-1 (Fujiwara, Aoki et al., 2005) and lysostaphin (Bardelang, Vankemmelbeke et al., 2009). The absence of each of these residues separately, especially both at the same time, blocks the catalytic activity of the enzyme determining its antibacterial properties. A construct with an incomplete active center cannot carry out the cleavage reaction of peptide bonds leading to cell wall disintegration and thus lysis of bacterial cells. It was demonstrated that the chimeras known in the state of the art are catalytically inactive.

### Example 8. Comparison of Chimera B activity with and without CPP sequences under different environmental conditions

Lytic activity studies against *S. aureus* under various environmental conditions were carried out for the Chimera B constructs produced according to Example 1 with and without CPP signal sequences.

The comparison of the activity was carried out for: i) Chimera B, ii) Chimera B with CPP (signal sequence RQIKIWFQNRRMKWKK **(SEQ ID No. 9)** attached at the N-terminus). The activity of the enzymes was compared under the following conditions.

### 8.1. Activity in glycine buffer

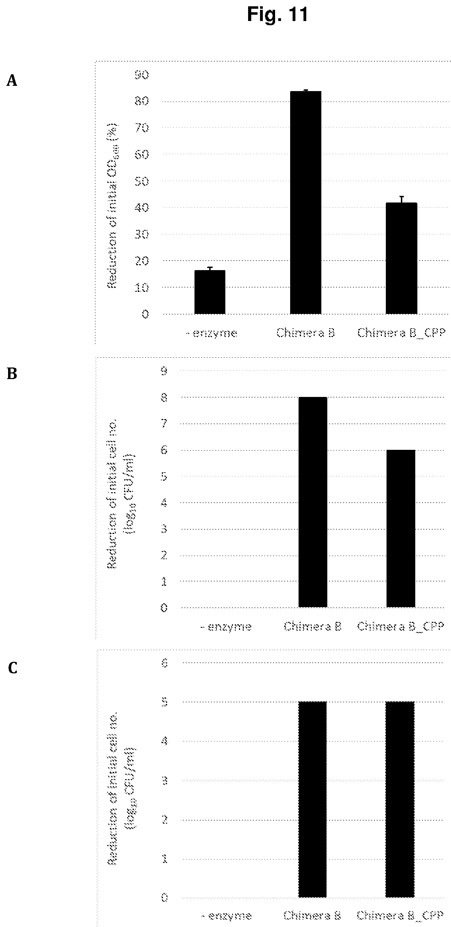

The lytic activity of the purified chimeric enzymes was monitored in the test of turbidity reduction of bacterial suspensions. Reference strain of *S. aureus* (NCTC 8325-4) was grown in Tryptic Soy Broth (TSB) medium at 37 °C and bacterial cells were collected at exponential growth phase, washed, and suspended in 50 mM glycine, pH 8.0, 100 mM NaCl buffer to OD₅₉₅ 1.0 (corresponding to 10⁸ CFU/ml). The activity of 500 nM of enzymes, observed as a decrease in OD₅₉₅, was monitored for 1 hour at room temperature and the values shown in the graph present the decrease in starting OD₅₉₅ after 1 hour (mean ± SD). The negative control was a sample incubated without the enzyme. The experiment was repeated 3 times in triplicates. The obtained mean results are shown in **Fig. 11A****.**

### 8.2. Activity in serum

The lytic activity of the chimeric enzymes was measured as a percentage of the reduction of the initial number of cells that were used to inoculate fetal bovine serum. The reference strain of *S*. *aureus* (NCTC 8325-4) was grown in Tryptic Soy Broth (TSB) medium at 37 °C, bacterial cells were collected at exponential growth phase. The serum was inoculated with 10⁸ CFU/ml and incubated with 500 nM of the specified enzyme for 3 hours at 37 °C. The number of bacterial cells was determined in samples collected after 3 hours by serial dilutions which were plated on TSB-agar plates. The plates were incubated at 37 °C for 18 hours and the result was presented as the log₁₀ decrease of the initial CFU/ml. The negative control was a sample incubated without the enzyme. The experiment was repeated 3 times in triplicates. The obtained mean results are shown in **Fig. 11B****.**

### 8.3. Activity in UHT milk

The lytic activity of the chimeric enzymes was measured as a percentage of the reduction of the initial number of cells that were used to inoculate 50% milk (diluted with 50 mM glycine, pH 8.0, 100 mM NaCl). The reference strain of *S. aureus* (NCTC 8325-4) was grown in Tryptic Soy Broth (TSB) medium at 37 °C and the bacterial cells were collected at exponential growth phase. The serum was inoculated with 10⁶ CFU/ml and incubated with 1 µM of the appropriate enzyme for 3 hours at 37 °C. The number of bacterial cells was determined in samples collected after 3 hours by serial dilutions which were plated on TSB-agar plates. The plates were incubated at 37 °C for 18 hours and the result was presented as the log₁₀ decrease of the initial CFU/ml. The negative control was a sample incubated without the enzyme. The experiment was repeated 3 times in triplicates. The obtained mean results are shown in **Fig. 11C****.** It was shown that the binding of the CPP domain does not adversely affect the activity of Chimera A, and particularly, its activity in milk and serum.

### Example 9. The use of the recombinant polypeptides for the in vivo treatment of infected wounds

### 9.1. Preparation of a composition of hydrogel wound dressings with Chimera A and B

The commercially available wound dressings (AquaGel, KIKGEL) were cut into 1 cm² pieces and spotted with a standard PBS buffer solution (negative control) or the protein solution of the Chimera A or B in PBS buffer (test sample). The solutions were dropped directly onto the hydrogel pieces: for the test sample, 100 nM enzyme solution at a volume of 100 µl; for the control, 100 µl of PBS buffer) and left until the solution was absorbed. The hydrogels prepared in this way were stored at 4 °C until they were applied to the wounds.

### 9.2 In vivo antibacterial and anti-inflammatory effect of the hydrogel wound dressing with the recombinant polypeptide

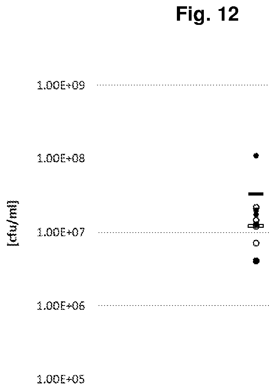

The overnight culture of the *S. aureus* FDA485 strain was centrifuged and resuspended in sterile PBS buffer. Bacterial cell count was adjusted to 1 in McFerland scale (corresponding to 1.61 x 10⁸ CFU/ml). Five female BL/6 mice were tested in each experimental variant. Surface wounds were generated by needle scratch, 50 µl of a bacterial suspension was applied directly onto the scratch and covered with a sterile cotton linter (ROTH). The infection developed for 24 h. After this time, the cotton linter was removed and a hydrogel wound dressing (without a Chimera - negative control or with the test sample - Chimera A or B) was applied to the infected wounds and placed directly on the wound. Every 12 hours the wound dressing was changed to a new one of the same type. 72 hours after the infection of the wound, all mice were sacrificed. The wound along with the surrounding skin was homogenized and suspended in 3 ml of PBS. Serial dilutions were made and plated on TSB-agar plates. Two serial dilutions were made for each variant. To calculate the mean value of bacterial infection, the grown *S. aureus* colonies were counted after the plates were incubated overnight at 37 °C. The obtained results were averaged for the results from at least three test plates. The obtained mean results are shown in **Fig. 12****.**

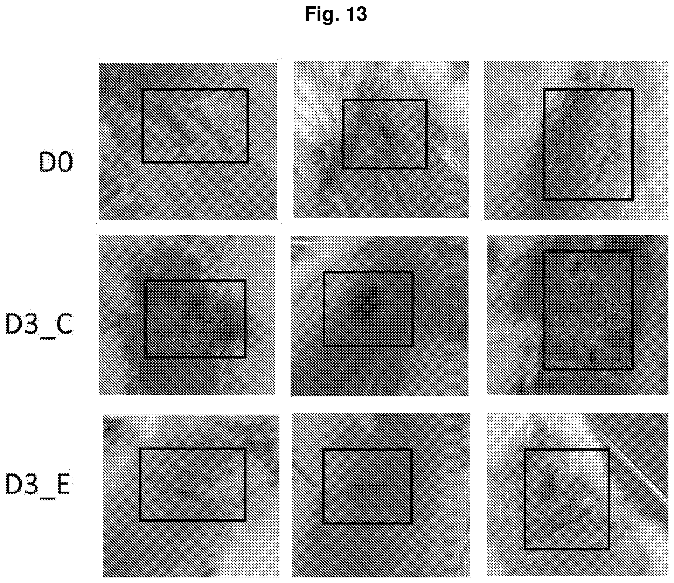

Photos of wounds during the experiment were presented in **Fig. 13****.** The top panel D0 shows an example of the appearance of wounds on three mice at the beginning of the experiment (day 0). Slightly reddened wounds on the surface of the skin with no hair are visible. The middle panel (D3_C) shows exemplary photos of the skin of control mice with infected wounds on which hydrogel wound dressings without enzyme were applied for three days. The development of a bacterial infection and inflammation caused by it, manifested by reddening of the skin around the wounds can be observed. The lower panel (D3_E) shows examples of the previously infected wounds onto which the enzyme-Chimera A hydrogel wound dressings were applied for three days. These wounds look very similar to the wounds from the beginning of the experiment (top panel D0). Wounds healing and inflammation subsiding due to the activity of the Chimera contained in the dressing is clearly visible. The inflammation observed in the control group (middle panel D3_C) has resolved, and there is no clearly visible redness around the wounds.

The performed *in vivo* tests showed a significant decrease in the number of *S. aureus* bacteria in wounds treated with the Chimera dressing. There was a 63% reduction in the number of bacterial cells (reduction by 2.1x10⁷ CFU/ml) in the treated wound compared to the untreated wound. For statistical verification, a two-way ANOVA analysis was performed on the values after logarithmic transformation. The difference between the negative control and the sample is statistically significant (p <0.01). The obtained mean results are shown in **Fig. 12****.**

The recombinant polypeptides for use according to the invention show antibacterial activity against *S. aureus* when delivered to an infected wound via a hydrogel, as well as an anti-inflammatory activity. The recombinant polypeptides for use according to the invention show *in vivo* activity as a medicine against staphylococci, and are particularly useful for the treatment of *S. aureus* infections.

### LITERATURE

Bardelang, P., M. Vankemmelbeke, Y. Zhang, H. Jarvis, E. Antoniadou, S. Rochette, N. R. Thomas, C. N. Penfold and R. James (2009). "Design of a polypeptide FRET substrate that facilitates study of the antimicrobial protease lysostaphin." Biochem J 418(3): 615-624.
Bowler et al. (2001). "Wound microbiology and associated approaches to wound management." Clin Microbiol Rev 14(2): 244-269.
Brusselaers et al. (2010). "Severe burn injury in Europe: a systematic review of the incidence, etiology, morbidity, and mortality." Crit Care 14(5): R188.
Fujiwara, T., S. Aoki, H. Komatsuzawa, T. Nishida, M. Ohara, H. Suginaka and M. Sugai (2005). "Mutation analysis of the histidine residues in the glycylglycine endopeptidase ALE-1." J Bacteriol 187(2): 480-487.
Hicks et al. (2014). "Trends and determinants of costs associated with the inpatient care of diabetic foot ulcers." J Vasc Surg 60(5): 1247-1254, 1254.e1241-1242.
Hop et al. (2014). "Costs of burn care: a systematic review." Wound Repair Regen 22(4): 436-450. Jagielska, E., O. Chojnacka and I. Sabala (2016). "LytM fusion with SH3b-like domain restore its activity in physiological conditions."
Karthikesalingam et al. (2010). "A systematic review of scoring systems for diabetic foot ulcers." Diabetic Medicine 27(5): 544-549.
Kruse and Edelman (2006). "Evaluation and Treatment of Diabetic Foot Ulcers." Clinical Diabetes 24(2): 91-93.
Malinowski and Smulski (2007). "Prevalence and prophylaxis of intramammary infections and mastitis in pregnant heifers." Zycie Weterynaryjne 82(6): 476-482.
Odintsov, S. G., I. Sabala, M. Marcyjaniak and M. Bochtler (2004). "Latent LytM at 1.3A resolution." J Mol Biol 335(3): 775-785.
Osipovitch, D. C. and K. E. Griswold (2015). "Fusion with a cell wall binding domain renders autolysin LytM a potent anti-Staphylococcus aureus agent." FEMS Microbiol Lett 362(2): 1-7.
Osipovitch DC, Therrien S, Griswold KE (2015). "Discovery of novel S. aureus autolysins and molecular engineering to enhance bacteriolytic activity". Appl Microbiol Biotechnol. 2015 Aug; 99(15): 6315-26.
Rodriguez-Rubio, L., B. Martinez, D. M. Donovan, P. Garcia and A. Rodriguez (2013). "Potential of the virion-associated peptidoglycan hydrolase HydH5 and its derivative fusion proteins in milk biopreservation." PLoS One 8(1): e54828.
Verbree, C. T., S. M. Datwyler, S. Meile, F. Eichenseher, D. M. Donovan, M. J. Loessner and M. Schmelcher (2018). "Corrected and Republished from: Identification of Peptidoglycan Hydrolase Constructs with Synergistic Staphylolytic Activity in Cow's Milk." Appl Environ Microbiol 84(1).

## Claims

1. A recombinant polypeptide comprising
(i) a LytM catalytic domain (LytMCD), comprising an amino acid sequence shown in SEQ ID NO: 1 or having at least 80% identity thereto, which retains the bacteriolytic activity against *Staphylococcus aureus* and wherein the zinc-coordinating amino acids in the active center are not changed and are His in position 26, Asp in position 30, His in position 109 of the SEQ ID NO: 1;
(ii) a lysostaphin domain, binding the cell wall of bacteria (LssCWT), comprising an amino acid sequence shown in SEQ ID NO: 2 or having at least 80% identity thereto;
(iii) from the LssCWT domain side, at the N or C terminus of the polypeptide, has an attached Cell-Penetrating Peptide (CPP) signal sequence directing to the inside of an eukaryotic cell;for use in the treatment of a disease and/or condition caused by a bacterium of the genus *Staphylococcus.*

2. The recombinant polypeptide for use according to claim 1, wherein the disease and/or condition is selected from the group consisting of acne, pimples, dermatitis, preferably atopic dermatitis, infections and/or injuries of a mucous membrane or of the skin, wounds, diabetic foot, ulcers, burns, and an inflammation of the mammary gland, inflammation of the udder, eye, ear, throat, sinuses or genitals.

3. The recombinant polypeptide for use according to claims 1-2, wherein the recombinant polypeptide is formulated for topical administration, preferably in the form of a cream, suspension, lotion, solution, ointment, gel, foam, transdermal patch, powder, paste, suspension, tape, pad, swab, wound dressing or compress.

4. The recombinant polypeptide for use according to claims 1-3, wherein the recombinant polypeptide is formulated for intramammary delivery, preferably for delivery to a nipple, teat, udder, preferably by infusion, rinsing or dipping,
preferably wherein the recombinant polypeptide is for delivery to a female human or non-human animal suffering from mastitis or at risk of developing mastitis, preferably a ruminant, more preferably a dairy cow.

5. A pharmaceutical or veterinary composition comprising a recombinant polypeptide comprising
(i) a LytM catalytic domain (LytMCD), comprising an amino acid sequence shown in SEQ ID NO: 1 or having at least 80% identity thereto, which retains the bacteriolytic activity against *Staphylococcus aureus,* and wherein the zinc-coordinating amino acids in the active center are not changed and are His in position 26, Asp in position 30, His in position 109 of the SEQ ID NO: 1;
(ii) a lysostaphin domain, binding the cell wall of bacteria (LssCWT), comprising an amino acid sequence shown in SEQ ID NO: 2 or having at least 80% identity thereto;
(iii) from the LssCWT domain side, at the N or C terminus of the polypeptide, has an attached Cell-Penetrating Peptide (CPP) signal sequence directing to the inside of an eukaryotic cell,and a pharmaceutically or veterinary acceptable excipient for use in the treatment of a disease and /or condition caused by a bacterium of the genus *Staphylococcus.*

6. The pharmaceutical or veterinary composition for use according to claim 5, wherein the disease and/or condition caused by bacterium of the genus *Staphylococcus* is selected from the group consisting of dermatitis, injuries and/or infections of a mucous membrane or of the skin, wounds, ulcers, inflammation of the mammary gland, inflammation of an eye, ear, throat, sinuses or genitals, preferably wherein the disease and/or condition is mastitis in ruminants and the composition is formulated for intramammary administration.

7. A use of a recombinant polypeptide comprising:
(i) a LytM catalytic domain (LytMCD), comprising an amino acid sequence shown in SEQ ID NO: 1 or having at least 80% identity thereto, which retains the bacteriolytic activity against *Staphylococcus aureus,* and wherein the zinc-coordinating amino acids in the active center are not changed and are His in position 26, Asp in position 30, His in position 109 of the SEQ ID NO: 1;
(ii) a cell wall binding lysostaphin domain, binding the cell wall of bacteria (LssCWT), comprising an amino acid sequence shown in SEQ ID NO: 2 or having at least 80% identity thereto;
(iii) from the LssCWT domain side, at the N or C terminus of the polypeptide, has an attached Cell-Penetrating Peptide (CPP) signal sequence directing to the inside of an eukaryotic cell,
as disinfectant, *in-vitro* antibacterial agent, *in-vitro* active agent in a cosmetic, care or hygienic composition for humans or animals against a bacterium of the genus *Staphylococcus.*

8. The use of a recombinant polypeptide according to claim 7, wherein when the polypeptide is used as the disinfectant, it is used for disinfecting surfaces, appliances and equipment, furniture, floors, particularly in hospitals, medical and dental offices, sports facilities, production lines, food factories and processing plants, kitchens and bathrooms.

9. The use of a recombinant polypeptide according to claims 7, wherein when the polypeptide is use as the *in-vitro* antibacterial agent in milk, serum, preferably in whole milk.

10. A cosmetic or care composition for cosmetic, care and hygienic use in humans or animals, wherein it comprises a recombinant polypeptide comprising
(i) a LytM catalytic domain (LytMCD), comprising an amino acid sequence shown in SEQ ID NO: 1 or having at least 80% identity thereto which retains the bacteriolytic activity against *Staphylococcus aureus,* and wherein the zinc-coordinating amino acids in the active center are not changed and are His in position 26, Asp in position 30, His in position 109 of the SEQ ID NO: 1;
(ii) a lysostaphin domain, binding the cell wall of bacteria (LssCWT), comprising an amino acid sequence shown in SEQ ID NO: 2 or having at least 80% identity thereto;
(iii) from the LssCWT domain side, at the N or C terminus of the polypeptide, has an attached Cell-Penetrating Peptide (CPP) signal sequence directing to the inside of an eukaryotic cell,
wherein the composition additionally comprises at least one carrier approved for care and hygienic used in humans or animals, and is intended for external use,
and wherein the recombinant polypeptide is used to limit the occurrence or to remove a bacteria of the genus *Staphylococcus* in the cosmetic or care composition.

11. The recombinant polypeptide for use according to claim 1, the pharmaceutical or veterinary composition comprising the recombinant polypeptide for use according to claim 5, the use of the recombinant polypeptide according to claim 7, the cosmetic or care composition comprising the recombinant polypeptide for use according to claim 10,
wherein the CCP sequence is selected from SEQ ID NO: 9-19.

12. A recombinant polypeptide comprising
(i) a LytM catalytic domain (LytMCD), comprising an amino acid sequence shown in SEQ ID NO: 1 or having at least 80% identity thereto which retains the bacteriolytic activity against *Staphylococcus aureus,* and wherein the zinc-coordinating amino acids in the active center are not changed and are His in position 26, Asp in position 30, His in position 109 of the SEQ ID NO: 1;
(ii) a lysostaphin domain, binding the cell wall of bacteria (LssCWT), comprising an amino acid sequence shown in SEQ ID NO: 2 or having at least 80% identity thereto,
wherein the recombinant polypeptide exhibits bacteriolytic activity in raw milk and/or serum, for use in the treatment of mastitis or prevention of developing mastitis in a cow, caused by a bacterium of the genus *Staphylococcus,* and
wherein the recombinant polypeptide is administered to the raw milk in the udder of the cow.

13. The recombinant polypeptide for use according to claim 12, **characterized in that** it further, from the LssCWT domain side, at the N or C terminus of the polypeptide, has an attached CPP signal sequence directing to the inside of an eukaryotic cell, preferably the signal sequence is selected from SEQ ID NO: 9-19.

14. The recombinant polypeptide for use according to claims 12-13, wherein it is in the form of pharmaceutical or veterinary composition additionally comprising pharmaceutically or veterinary acceptable excipient.

15. The recombinant polypeptide for use according to claims 12-14, wherein the administration is performed by infusion, rinsing or dipping.

16. A use of a recombinant polypeptide comprising:
(i) a LytM catalytic domain (LytMCD), comprising an amino acid sequence shown in SEQ ID NO: 1 or having at least 80% identity thereto which retains the bacteriolytic activity against *Staphylococcus aureus,* and wherein the zinc-coordinating amino acids in the active center are not changed and are His in position 26, Asp in position 30, His in position 109 of the SEQ ID NO: 1;
(ii) a cell wall binding lysostaphin domain, binding the cell wall of bacteria (LssCWT), comprising an amino acid sequence shown in SEQ ID NO: 2 or having at least 80% identity thereto;
as *in-vitro* antibacterial agent against a bacterium of the genus *Staphylococcus* in raw milk.

17. The recombinant polypeptide for use according to claim 1, the pharmaceutical or veterinary composition comprising the recombinant polypeptide for use according to claim 5, the use of the recombinant polypeptide according to claim 7, the cosmetic or care composition for cosmetic, care and hygienic use in humans or animals according to claim 10, the recombinant polypeptide for use in the treatment of mastitis or prevention of developing mastitis according to claim 12, the use of recombinant polypeptide as *in-vitro* antibacterial agent according to claim 16,
wherein the LytM catalytic domain consists of the amino acid sequence shown in SEQ ID NO: 1.

18. The recombinant polypeptide for use according to claim 1, the pharmaceutical or veterinary composition comprising the recombinant polypeptide for use according to claim 5, the use of the recombinant polypeptide according to claim 7, the cosmetic or care composition for cosmetic, care and hygienic use in humans or animals according to claim 10, the recombinant polypeptide for use in the treatment of mastitis or prevention of developing mastitis according to claim 12, the use of recombinant polypeptide as *in-vitro* antibacterial agent according to claim 16,
wherein the lysostaphin cell wall binding domain consists of the amino acid sequence shown in SEQ ID NO: 2.

19. The recombinant polypeptide for use according to claim 1, the pharmaceutical or veterinary composition comprising the recombinant polypeptide for use according to claim 5, the use of the recombinant polypeptide according to claim 7, the cosmetic or care composition for cosmetic, care and hygienic use in humans or animals according to claim 10, the recombinant polypeptide for use in the treatment of mastitis or prevention of developing mastitis according to claim 12, the use of recombinant polypeptide as *in-vitro* antibacterial agent according to claim 16,
wherein it further comprises a peptide linker, linking the LytM catalytic domain and the lysostaphin cell wall binding domain,
wherein the peptide linker preferably comprises the amino acid sequence shown in SEQ ID NO: 3 or SEQ ID NO: 4, preferably, peptide linker consists of the sequence SEQ ID NO: 3.

20. The recombinant polypeptide for use according to claim 1, the pharmaceutical or veterinary composition comprising the recombinant polypeptide for use according to claim 5, the use of the recombinant polypeptide according to claim 7, the cosmetic or care composition for cosmetic, care and hygienic use in humans or animals according to claim 10, the recombinant polypeptide for use in the treatment of mastitis or prevention of developing mastitis according to claim 12, the use of recombinant polypeptide as *in-vitro* antibacterial agent according to claim 16,
wherein the recombinant polypeptide comprises the sequence SEQ ID NO: 5, or is encoded by a nucleotide sequence comprising SEQ ID NO: 6;
or wherein the recombinant polypeptide comprises the sequence SEQ ID NO: 7, or is encoded by a nucleotide sequence comprising SEQ ID NO: 8.

21. The recombinant polypeptide for use according to claim 1, the pharmaceutical or veterinary composition comprising the recombinant polypeptide for use according to claim 5, the use of the recombinant polypeptide according to claim 7, the cosmetic or care composition for cosmetic, care and hygienic use in humans or animals according to claim 10, the recombinant polypeptide for use in the treatment of mastitis or prevention of developing mastitis according to claim 12, the use of recombinant polypeptide as *in-vitro* antibacterial agent according to claim 16,
wherein the bacteria of the genus *Staphylococcus* is *Staphylococcus aureus.*

## Patentansprüche

1. Rekombinantes Polypeptid, umfassend
(i) eine katalytische Domäne von LytM (LytMCD), umfassend eine in SEQ ID NO: 1 gezeigte Aminosäuresequenz oder mit einer Identität von mindestens 80 % dazu, die die bakteriolytische Aktivität gegen *Staphylococcus aureus* beibehält, und wobei die Zink-koordinierenden Aminosäuren im aktiven Zentrum nicht verändert sind und His an Position 26, Asp an Position 30, His an Position 109 der SEQ ID NO: 1 sind;
(ii) eine Domäne von Lysostaphin, die die Zellwand von Bakterien (LssCWT) bindet, umfassend eine in SEQ ID NO: 2 gezeigte Aminosäuresequenz oder mit einer Identität von mindestens 80 % dazu;
(iii) von der Seite der Domäne von LssCWT, am N- oder C-Terminus des Polypeptids, eine anhängende Signalsequenz des Zellpenetrierenden Peptids (CPP) aufweist, die in das Innere einer eukaryotischen Zelle weist; zur Verwendung bei der Behandlung einer Krankheit und/oder eines Zustands, die/der durch ein Bakterium der Gattung *Staphylococcus* verursacht wird.

2. Rekombinantes Polypeptid zur Verwendung gemäß Anspruch 1, wobei die Krankheit und/oder der Zustand aus der Gruppe bestehend aus Akne, Pickeln, Dermatitis, vorzugsweise atopischer Dermatitis, Infektionen und/oder Verletzungen einer Schleimhaut oder der Haut, Wunden, diabetischem Fuß, Geschwüren, Verbrennungen und einer Entzündung der Brustdrüse, einer Entzündung des Euters, des Auges, des Ohrs, des Rachens, der Nasennebenhöhlen oder der Genitalien ausgewählt ist.

3. Rekombinantes Polypeptid zur Verwendung gemäß den Ansprüchen 1-2, wobei das rekombinante Polypeptid zur topischen Verabreichung formuliert ist, vorzugsweise in Form einer Creme, einer Suspension, einer Lotion, einer Lösung, einer Salbe, eines Gels, eines Schaums, eines transdermalen Pflasters, eines Pulvers, einer Paste, einer Suspension, eines Bandes, eines Pads, eines Tupfers, eines Wundverbands oder einer Kompresse.

4. Rekombinantes Polypeptid zur Verwendung gemäß den Ansprüchen 1-3, wobei das rekombinante Polypeptid zur intramammären Abgabe formuliert ist, vorzugsweise zur Abgabe an eine Brustwarze, eine Zitze, einen Euter, vorzugsweise durch Infusion, Spülen oder Eintauchen,
vorzugsweise wobei das rekombinante Polypeptid zur Abgabe an ein weibliches menschliches oder nichtmenschliches Tier bestimmt ist, das an Mastitis leidet oder bei dem das Risiko besteht, eine Mastitis zu entwickeln, vorzugsweise ein Wiederkäuer, besonders bevorzugt eine Milchkuh.

5. Pharmazeutische oder veterinärmedizinische Zusammensetzung, umfassend ein rekombinantes Polypeptid, umfassend:
(i) eine katalytische Domäne von LytM (LytMCD), umfassend eine in SEQ ID NO: 1 gezeigte Aminosäuresequenz oder mit einer Identität von mindestens 80 % dazu, die die bakteriolytische Aktivität gegen *Staphylococcus aureus* beibehält, und wobei die Zink-koordinierenden Aminosäuren im aktiven Zentrum nicht verändert sind und His an Position 26, Asp an Position 30, His an Position 109 der SEQ ID NO: 1 sind;
(ii) eine Domäne von Lysostaphin, die die Zellwand von Bakterien (LssCWT) bindet, umfassend eine in SEQ ID NO: 2 gezeigte Aminosäuresequenz oder mit einer Identität von mindestens 80 % dazu;
(iii) von der Seite der Domäne von LssCWT, am N- oder C-Terminus des Polypeptids, eine anhängende Signalsequenz des Zellpenetrierenden Peptids (CPP) aufweist, die in das Innere einer eukaryotischen Zelle weist, und ein pharmazeutisch oder veterinärmedizinisch akzeptables Hilfsmittel zur Verwendung bei der Behandlung einer Krankheit und/oder eines Zustands, die/der durch ein Bakterium der Gattung *Staphylococcus* verursacht wird.

6. Pharmazeutische oder veterinärmedizinische Zusammensetzung zur Verwendung gemäß Anspruch 5, wobei die Krankheit und/oder der Zustand, die/der durch ein Bakterium der Gattung *Staphylococcus* verursacht wird, aus der Gruppe bestehend aus Dermatitis, Verletzungen und/oder Infektionen einer Schleimhaut oder der Haut, Wunden, Geschwüren, einer Entzündung der Brustdrüse, einer Entzündung eines Auges, Ohres, des Rachens, der Nasennebenhöhlen oder der Genitalien ausgewählt ist, wobei vorzugsweise die Krankheit und/oder der Zustand eine Mastitis bei Wiederkäuern ist und die Zusammensetzung zur intramammären Verabreichung formuliert ist.

7. Verwendung eines rekombinanten Polypeptids, umfassend:
(i) eine katalytische Domäne von LytM (LytMCD), umfassend eine in SEQ ID NO: 1 gezeigte Aminosäuresequenz oder mit einer Identität von mindestens 80 % dazu, die die bakteriolytische Aktivität gegen *Staphylococcus aureus* beibehält, und wobei die Zink-koordinierenden Aminosäuren im aktiven Zentrum nicht verändert sind und His an Position 26, Asp an Position 30, His an Position 109 der SEQ ID NO: 1 sind;
(ii) eine Zellwand-bindende Domäne von Lysostaphin, die die Zellwand von Bakterien (LssCWT) bindet, umfassend eine in SEQ ID NO: 2 gezeigte Aminosäuresequenz oder mit einer Identität von mindestens 80 % dazu;
(iii) von der Seite der Domäne von LssCWT, am N- oder C-Terminus des Polypeptids, eine anhängende Signalsequenz des Zellpenetrierenden Peptids (CPP) aufweist, die in das Innere einer eukaryotischen Zelle weist,
als Desinfektionsmittel, *in-vitro*-Antibakterium, *in-vitro*-Wirkstoff in einer kosmetischen, pflegenden oder hygienischen Zusammensetzung für Menschen oder Tiere gegen ein Bakterium der Gattung *Staphylococcus.*

8. Verwendung eines rekombinanten Polypeptids gemäß Anspruch 7, wobei, wenn das Polypeptid als Desinfektionsmittel verwendet wird, es zur Desinfektion von Oberflächen, Geräten und Ausrüstungen, Möbeln, Fußböden, insbesondere in Krankenhäusern, medizinischen und zahnärztlichen Praxen, Sportanlagen, Produktionslinien, Lebensmittel- und Verarbeitungsanlagen, Küchen und Bädern verwendet wird.

9. Verwendung eines rekombinanten Polypeptids gemäß Anspruch 7, wobei das Polypeptid als *in-vitro*-Antibakterium in Milch, Serum, vorzugsweise in Vollmilch verwendet wird.

10. Kosmetische oder pflegende Zusammensetzung für die kosmetische, pflegende und hygienische Anwendung bei Menschen oder Tieren, wobei sie ein rekombinantes Polypeptid umfasst, umfassend
(i) eine katalytische Domäne von LytM (LytMCD), umfassend eine in SEQ ID NO: 1 gezeigte Aminosäuresequenz oder mit einer Identität von mindestens 80 % dazu, die die bakteriolytische Aktivität gegen *Staphylococcus aureus* beibehält, und wobei die Zink-koordinierenden Aminosäuren im aktiven Zentrum nicht verändert sind und His an Position 26, Asp an Position 30, His an Position 109 der SEQ ID NO: 1 sind;
(ii) eine Domäne von Lysostaphin, die die Zellwand von Bakterien (LssCWT) bindet, umfassend eine in SEQ ID NO: 2 gezeigte Aminosäuresequenz oder mit einer Identität von mindestens 80 % dazu;
(iii) von der Seite der Domäne von LssCWT, am N- oder C-Terminus des Polypeptids, eine anhängende Signalsequenz des Zellpenetrierenden Peptids (CPP) aufweist, die in das Innere einer eukaryotischen Zelle weist,
wobei die Zusammensetzung zusätzlich mindestens einen Träger umfasst, der für pflegende und hygienische Anwendungen bei Menschen oder Tieren zugelassen ist, und für die äußere Anwendung bestimmt ist,
und wobei das rekombinante Polypeptid verwendet wird, um das Auftreten eines Bakteriums der Gattung *Staphylococcus* in der kosmetischen oder pflegenden Zusammensetzung zu begrenzen oder zu entfernen.

11. Rekombinantes Polypeptid zur Verwendung gemäß Anspruch 1, pharmazeutische oder veterinärmedizinische Zusammensetzung, umfassend das rekombinante Polypeptid zur Verwendung gemäß Anspruch 5, Verwendung des rekombinanten Polypeptids gemäß Anspruch 7, kosmetische oder pflegende Zusammensetzung, umfassend das rekombinante Polypeptid zur Verwendung gemäß Anspruch 10,
wobei die CCP-Sequenz aus SEQ ID NO: 9-19 ausgewählt ist.

12. Rekombinantes Polypeptid, umfassend
(i) eine katalytische Domäne von LytM (LytMCD), umfassend eine in SEQ ID NO: 1 gezeigte Aminosäuresequenz oder mit einer Identität von mindestens 80 % dazu, die die bakteriolytische Aktivität gegen *Staphylococcus aureus* beibehält, und wobei die Zink-koordinierenden Aminosäuren im aktiven Zentrum nicht verändert sind und His an Position 26, Asp an Position 30, His an Position 109 der SEQ ID NO: 1 sind;
(ii) eine Domäne von Lysostaphin, die die Zellwand von Bakterien (LssCWT) bindet, umfassend eine in SEQ ID NO: 2 gezeigte Aminosäuresequenz oder mit einer Identität von mindestens 80 % dazu,
wobei das rekombinante Polypeptid bakteriolytische Aktivität in Rohmilch und/oder Serum aufweist,
zur Verwendung bei der Behandlung von Mastitis oder zur Verhinderung der Entwicklung von Mastitis bei einer Kuh, die durch ein Bakterium der Gattung *Staphylococcus* verursacht wird, und
wobei das rekombinante Polypeptid der Rohmilch im Euter der Kuh verabreicht wird.

13. Rekombinantes Polypeptid zur Verwendung gemäß Anspruch 12, **dadurch gekennzeichnet, dass** es zusätzlich von der Seite der Domäne von LssCWT, am N- oder C-Terminus des Polypeptids, eine anhängende CPP-Signalsequenz aufweist, die in das Innere einer eukaryotischen Zelle weist, vorzugsweise wobei die Signalsequenz aus SEQ ID NO: 9-19 ausgewählt ist.

14. Rekombinantes Polypeptid zur Verwendung gemäß den Ansprüchen 12-13, wobei es in Form einer pharmazeutischen oder veterinärmedizinischen Zusammensetzung vorliegt, zusätzlich umfassend ein pharmazeutisch oder veterinärmedizinisch akzeptables Hilfsmittel.

15. Rekombinantes Polypeptid zur Verwendung gemäß den Ansprüchen 12-14, wobei die Verabreichung durch Infusion, Spülen oder Eintauchen erfolgt.

16. Verwendung eines rekombinanten Polypeptids, umfassend:
(i) eine katalytische Domäne von LytM (LytMCD), umfassend eine in SEQ ID NO: 1 gezeigte Aminosäuresequenz oder mit einer Identität von mindestens 80 % Identität dazu, die die bakteriolytische Aktivität gegen *Staphylococcus aureus* beibehält, und wobei die Zink-koordinierenden Aminosäuren im aktiven Zentrum nicht verändert sind und His an Position 26, Asp an Position 30, His an Position 109 der SEQ ID NO: 1 sind;
(ii) eine Zellwand-bindende Domäne von Lysostaphin, die die Zellwand von Bakterien (LssCWT) bindet, umfassend eine in SEQ ID NO: 2 gezeigte Aminosäuresequenz oder mit einer Identität von mindestens 80 % dazu;
als in-vitro-Antibakterium gegen ein Bakterium der Gattung *Staphylococcus* in Rohmilch.

17. Rekombinantes Polypeptid zur Verwendung gemäß Anspruch 1, pharmazeutische oder veterinärmedizinische Zusammensetzung, umfassend das rekombinante Polypeptid zur Verwendung gemäß Anspruch 5, Verwendung des rekombinanten Polypeptids gemäß Anspruch 7, kosmetische oder pflegende Zusammensetzung zur kosmetischen, pflegenden und hygienischen Anwendung bei Menschen oder Tieren gemäß Anspruch 10, rekombinantes Polypeptid zur Verwendung bei der Behandlung von Mastitis oder zur Verhinderung der Entwicklung von Mastitis gemäß Anspruch 12, Verwendung des rekombinanten Polypeptids als *in-vitro-*Antibakterium gemäß Anspruch 16,
wobei die katalytische Domäne von LytM aus der in SEQ ID NO: 1 gezeigten Aminosäuresequenz besteht.

18. Rekombinantes Polypeptid zur Verwendung gemäß Anspruch 1, pharmazeutische oder veterinärmedizinische Zusammensetzung, umfassend das rekombinante Polypeptid zur Verwendung gemäß Anspruch 5, Verwendung des rekombinanten Polypeptids gemäß Anspruch 7, kosmetische oder pflegende Zusammensetzung zur kosmetischen, pflegenden und hygienischen Anwendung bei Menschen oder Tieren gemäß Anspruch 10, rekombinantes Polypeptid zur Verwendung bei der Behandlung von Mastitis oder zur Verhinderung der Entwicklung von Mastitis gemäß Anspruch 12, Verwendung des rekombinanten Polypeptids als *in-vitro-*Antibakterium gemäß Anspruch 16,
wobei die Zellwand-bindende Domäne von Lysostaphin aus der in SEQ ID NO: 2 gezeigten Aminosäuresequenz besteht.

19. Rekombinantes Polypeptid zur Verwendung gemäß Anspruch 1, pharmazeutische oder veterinärmedizinische Zusammensetzung, umfassend das rekombinante Polypeptid zur Verwendung gemäß Anspruch 5, Verwendung des rekombinanten Polypeptids gemäß Anspruch 7, kosmetische oder pflegende Zusammensetzung zur kosmetischen, pflegenden und hygienischen Anwendung bei Menschen oder Tieren gemäß Anspruch 10, rekombinantes Polypeptid zur Verwendung bei der Behandlung von Mastitis oder zur Verhinderung der Entwicklung von Mastitis gemäß Anspruch 12, Verwendung des rekombinanten Polypeptids als *in-vitro-*Antibakterium gemäß Anspruch 16,
wobei es ferner einen Peptid-Linker umfasst, der die katalytische Domäne von LytM und die Zellwand-bindende Domäne von Lysostaphin verbindet,
wobei der Peptid-Linker vorzugsweise die in SEQ ID NO: 3 oder SEQ ID NO: 4 gezeigte Aminosäuresequenz umfasst, vorzugsweise wobei der Peptid-Linker aus der Sequenz SEQ ID NO: 3 besteht.

20. Rekombinantes Polypeptid zur Verwendung gemäß Anspruch 1, pharmazeutische oder veterinärmedizinische Zusammensetzung, umfassend das rekombinante Polypeptid zur Verwendung gemäß Anspruch 5, Verwendung des rekombinanten Polypeptids gemäß Anspruch 7, kosmetische oder pflegende Zusammensetzung zur kosmetischen, pflegenden und hygienischen Anwendung bei Menschen oder Tieren gemäß Anspruch 10, rekombinantes Polypeptid zur Verwendung bei der Behandlung von Mastitis oder zur Verhinderung der Entwicklung von Mastitis gemäß Anspruch 12, Verwendung des rekombinanten Polypeptids als *in-vitro-*Antibakterium gemäß Anspruch 16,
wobei das rekombinante Polypeptid die Sequenz SEQ ID NO: 5 umfasst, oder durch eine Nukleotidsequenz kodiert wird, die SEQ ID NO: 6 umfasst;
oder wobei das rekombinante Polypeptid die Sequenz SEQ ID NO: 7 umfasst, oder durch eine Nukleotidsequenz kodiert wird, die SEQ ID NO: 8 umfasst.

21. Rekombinantes Polypeptid zur Verwendung gemäß Anspruch 1, pharmazeutische oder veterinärmedizinische Zusammensetzung, umfassend das rekombinante Polypeptid zur Verwendung gemäß Anspruch 5, Verwendung des rekombinanten Polypeptids gemäß Anspruch 7, kosmetische oder pflegende Zusammensetzung zur kosmetischen, pflegenden und hygienischen Anwendung bei Menschen oder Tieren gemäß Anspruch 10, rekombinantes Polypeptid zur Verwendung bei der Behandlung von Mastitis oder zur Verhinderung der Entwicklung von Mastitis gemäß Anspruch 12, Verwendung des rekombinanten Polypeptids als *in-vitro-*Antibakterium gemäß Anspruch 16,
wobei die Bakterien der Gattung *Staphylococcus Staphylococcus aureus* sind.

## Revendications

1. Polypeptide recombinant comprenant
(i) un domaine catalytique de LytM (LytMCD), comprenant une séquence d'acides aminés représentée dans SEQ ID NO : 1 ou ayant au moins 80 % d'identité avec celle-ci, qui conserve l'activité bactériolytique contre *Staphylococcus aureus,* et dans lequel les acides aminés coordonnant le zinc au centre actif ne sont pas modifiés et sont His en position 26, ASP en position 30, His en position 109 de la SEQ ID NO : 1 ;
(ii) un domaine de lysostaphine, liant la paroi cellulaire de bactéries (LssCWT), comprenant une séquence d'acides aminés représentée dans SEQ ID NO : 2 ou ayant au moins 80 % d'identité avec celle-ci ;
(iii) du côté du domaine de LssCWT, à l'extrémité N- ou C-terminale du polypeptide, a une séquence signal de peptide pénétrant dans les cellules (CPP) attachée dirigeant vers l'intérieur d'une cellule eucaryote ; destiné à être utilisé dans le traitement d'une maladie et/ou d'un état induit par une bactérie du genre *Staphylococcus.*

2. Polypeptide recombinant destiné à être utilisé selon la revendication 1, dans lequel la maladie et/ou l'état est choisi dans le groupe constitué par l'acné, les boutons, la dermatite, de préférence la dermatite atopique, les infections et/ou blessures d'une muqueuse ou de la peau, les plaies, le pied diabétique, les ulcères, les brûlures et une inflammation de la glande mammaire, une inflammation de la mamelle, de l'œil, de l'oreille, de la gorge, des sinus ou des organes génitaux.

3. Polypeptide recombinant destiné à être utilisé selon les revendications 1-2, dans lequel le polypeptide recombinant est formulé pour une administration topique, de préférence sous la forme d'une crème, d'une suspension, d'une lotion, d'une solution, d'un onguent, d'un gel, d'une mousse, d'un timbre transdermique, d'une poudre, d'une pâte, d'une suspension, d'un ruban adhésif, d'un tampon, d'un écouvillon, d'un pansement ou d'une compresse.

4. Polypeptide recombinant destiné à être utilisé selon les revendications 1-3, dans lequel le polypeptide recombinant est formulé pour une administration intramammaire, de préférence pour une administration à un mamelon, un trayon, une mamelle, de préférence par perfusion, rinçage ou trempage,
de préférence dans lequel le polypeptide recombinant est destiné à être administré à un animal humain ou non-humain femelle souffrant d'une mammite ou présentant un risque de développer une mammite, de préférence un ruminant, de préférence une vache laitière.

5. Composition pharmaceutique ou vétérinaire comprenant un polypeptide recombinant comprenant
(i) un domaine catalytique de LytM (LytMCD), comprenant une séquence d'acides aminés représentée dans SEQ ID NO : 1 ou ayant au moins 80 % d'identité avec celle-ci, qui conserve l'activité bactériolytique contre *Staphylococcus aureus,* et dans lequel les acides aminés coordonnant le zinc au centre actif ne sont pas modifiés et sont His en position 26, ASP en position 30, His en position 109 de la SEQ ID NO : 1 ;
(ii) un domaine de lysostaphine, liant la paroi cellulaire de bactéries (LssCWT), comprenant une séquence d'acides aminés représentée dans SEQ ID NO : 2 ou ayant au moins 80 % d'identité avec celle-ci ;
(iii) du côté du domaine de LssCWT, à l'extrémité N- ou C-terminale du polypeptide, a une séquence signal de peptide pénétrant dans les cellules (CPP) attachée dirigeant vers l'intérieur d'une cellule eucaryote, et un excipient pharmaceutiquement ou vétérinairement acceptable destiné à être utilisé dans le traitement d'une maladie et/ou d'un état induit par une bactérie du genre *Staphylococcus.*

6. Composition pharmaceutique ou vétérinaire destinée à être utilisée selon la revendication 5, dans laquelle la maladie et/ou l'état induit par une bactérie du genre *Staphylococcus* est choisi dans le groupe constitué par la dermatite, les blessures et/ou les infections d'une muqueuse ou de la peau, les plaies, les ulcères, l'inflammation de la glande mammaire, l'inflammation d'un œil, d'une oreille, de la gorge, des sinus ou des organes génitaux, de préférence dans laquelle la maladie et/ou l'état est une mammite chez les ruminants et la composition est formulée pour une administration intramammaire.

7. Utilisation d'un polypeptide recombinant comprenant :
(i) un domaine catalytique de LytM (LytMCD), comprenant une séquence d'acides aminés représentée dans SEQ ID NO : 1 ou ayant au moins 80 % d'identité avec celle-ci, qui conserve l'activité bactériolytique contre *Staphylococcus aureus,* et dans lequel les acides aminés coordonnant le zinc au centre actif ne sont pas modifiés et sont His en position 26, ASP en position 30, His en position 109 de la SEQ ID NO : 1 ;
(ii) un domaine de liaison à la paroi cellulaire de lysostaphine, liant la paroi cellulaire de bactéries (LssCWT), comprenant une séquence d'acides aminés représentée dans SEQ ID NO : 2 ou ayant au moins 80 % d'identité avec celle-ci ;
(iii) du côté du domaine de LssCWT, à l'extrémité N- ou C-terminale du polypeptide, a une séquence signal de peptide pénétrant dans les cellules (CPP) attachée dirigeant vers l'intérieur d'une cellule eucaryote,
en tant que désinfectant, agent antibactérien *in vitro,* agent actif *in vitro* dans une composition cosmétique, de soin ou hygiénique pour les humains ou les animaux contre une bactérie du genre *Staphylococcus.*

8. Utilisation d'un polypeptide recombinant selon la revendication 7, dans laquelle, lorsque le polypeptide est utilisé en tant que désinfectant, il est utilisé pour désinfecter des surfaces, des appareils et des équipements, du mobilier, des sols, en particulier dans des hôpitaux, des cabinets médicaux et dentaires, des installations sportives, des chaînes de production, des usines alimentaires et des installations de transformation, des cuisines et des salles de bains.

9. Utilisation d'un polypeptide recombinant selon la revendication 7, dans laquelle le polypeptide est utilisé tant qu'un agent antibactérien *in vitro* dans le lait, le sérum, de préférence dans le lait entier.

10. Composition cosmétique ou de soin à usage cosmétique, de soin et hygiénique chez l'homme ou l'animal, dans laquelle elle comprend un polypeptide recombinant comprenant
(i) un domaine catalytique de LytM (LytMCD), comprenant une séquence d'acides aminés représentée dans SEQ ID NO : 1 ou ayant au moins 80 % d'identité avec celle-ci, qui conserve l'activité bactériolytique contre *Staphylococcus aureus,* et dans lequel les acides aminés coordonnant le zinc au centre actif ne sont pas modifiés et sont His en position 26, ASP en position 30, His en position 109 de la SEQ ID NO : 1 ;
(ii) un domaine de lysostaphine, liant la paroi cellulaire de bactéries (LssCWT), comprenant une séquence d'acides aminés représentée dans SEQ ID NO : 2 ou ayant au moins 80 % d'identité avec celle-ci ;
(iii) du côté du domaine de LssCWT, à l'extrémité N- ou C-terminale du polypeptide, a une séquence signal de peptide pénétrant dans les cellules (CPP) attachée dirigeant vers l'intérieur d'une cellule eucaryote,
dans laquelle la composition comprend en outre au moins un support approuvé pour des usages de soins et d'hygiène chez l'homme ou l'animal, et est destinée à une utilisation externe,
et dans laquelle le polypeptide recombinant est utilisé pour limiter l'apparition ou pour éliminer une bactérie du genre *Staphylococcus* dans la composition cosmétique ou de soin.

11. Polypeptide recombinant destiné à être utilisé selon la revendication 1, composition pharmaceutique ou vétérinaire comprenant le polypeptide recombinant destiné à être utilisé selon la revendication 5, utilisation du polypeptide recombinant selon la revendication 7, composition cosmétique ou de soin comprenant le polypeptide recombinant destiné à être utilisé selon la revendication 10,
dans lesquelles la séquence CCP est sélectionnée parmi SEQ ID NO : 9-19.

12. Polypeptide recombinant comprenant
(i) un domaine catalytique de LytM (LytMCD), comprenant une séquence d'acides aminés représentée dans SEQ ID NO : 1 ou ayant au moins 80 % d'identité avec celle-ci, qui conserve l'activité bactériolytique contre *Staphylococcus aureus,* et dans lequel les acides aminés coordonnant le zinc au centre actif ne sont pas modifiés et sont His en position 26, ASP en position 30, His en position 109 de la SEQ ID NO : 1 ;
(ii) un domaine de lysostaphine, liant la paroi cellulaire de bactéries (LssCWT), comprenant une séquence d'acides aminés représentée dans SEQ ID NO : 2 ou ayant au moins 80 % d'identité avec celle-ci ;
dans lequel le polypeptide recombinant présente une activité bactériolytique dans le lait cru et/ou le sérum,
destiné à être utilisé dans le traitement de la mammite ou la prévention du développement de la mammite chez une vache, induite par une bactérie du genre *Staphylococcus,* et
dans lequel le polypeptide recombinant est administré au lait cru dans la mamelle de la vache.

13. Polypeptide recombinant destiné à être utilisé selon la revendication 12, **caractérisé en ce qu'**il présente en outre, du côté du domaine de LssCWT, à l'extrémité N- ou C-terminale du polypeptide, une séquence signal CPP attachée dirigeant vers l'intérieur d'une cellule eucaryote, de préférence la séquence signal est sélectionnée parmi SEQ ID NO : 9-19.

14. Polypeptide recombinant destiné à être utilisé selon les revendications 12-13, dans lequel il se présente sous la forme d'une composition pharmaceutique ou vétérinaire comprenant en outre un excipient pharmaceutiquement ou vétérinairement acceptable.

15. Polypeptide recombinant destiné à être utilisé selon les revendications 12-14, dans lequel l'administration est effectuée par perfusion, rinçage ou trempage.

16. Utilisation d'un polypeptide recombinant comprenant :
(i) un domaine catalytique de LytM (LytMCD), comprenant une séquence d'acides aminés représentée dans SEQ ID NO : 1 ou ayant au moins 80 % d'identité avec celle-ci, qui conserve l'activité bactériolytique contre *Staphylococcus aureus,* et dans lequel les acides aminés coordonnant le zinc au centre actif ne sont pas modifiés et sont His en position 26, ASP en position 30, His en position 109 de la SEQ ID NO : 1 ;
(ii) un domaine de liaison à la paroi cellulaire de lysostaphine, liant la paroi cellulaire de bactéries (LssCWT), comprenant une séquence d'acides aminés représentée dans SEQ ID NO : 2 ou ayant au moins 80 % d'identité avec celle-ci ;
en tant qu'agent antibactérien *in vitro* contre une bactérie du genre *Staphylococcus* dans le lait cru.

17. Polypeptide recombinant destiné à être utilisé selon la revendication 1, composition pharmaceutique ou vétérinaire comprenant le polypeptide recombinant destiné à être utilisé selon la revendication 5, utilisation du polypeptide recombinant selon la revendication 7, composition cosmétique ou de soin à usage cosmétique, de soin et hygiénique chez l'homme ou l'animal selon la revendication 10, polypeptide recombinant destiné à être utilisé dans le traitement de la mammite ou la prévention du développement de la mammite selon la revendication 12, utilisation du polypeptide recombinant en tant qu'agent antibactérien *in vitro* selon la revendication 16,
dans lesquelles le domaine catalytique de LytM est constitué de la séquence d'acides aminés représentée dans SEQ ID NO : 1.

18. Polypeptide recombinant destiné à être utilisé selon la revendication 1, composition pharmaceutique ou vétérinaire comprenant le polypeptide recombinant destiné à être utilisé selon la revendication 5, utilisation du polypeptide recombinant selon la revendication 7, composition cosmétique ou de soin à usage cosmétique, de soin et hygiénique chez l'homme ou l'animal selon la revendication 10, polypeptide recombinant destiné à être utilisé dans le traitement de la mammite ou la prévention du développement de la mammite selon la revendication 12, utilisation du polypeptide recombinant en tant qu'agent antibactérien *in vitro* selon la revendication 16,
dans lesquelles le domaine de liaison à la paroi cellulaire de lysostaphine est constitué de la séquence d'acides aminés représentée dans SEQ ID NO : 2.

19. Polypeptide recombinant destiné à être utilisé selon la revendication 1, composition pharmaceutique ou vétérinaire comprenant le polypeptide recombinant destiné à être utilisé selon la revendication 5, utilisation du polypeptide recombinant selon la revendication 7, composition cosmétique ou de soin à usage cosmétique, de soin et hygiénique chez l'homme ou l'animal selon la revendication 10, polypeptide recombinant destiné à être utilisé dans le traitement de la mammite ou la prévention du développement de la mammite selon la revendication 12, utilisation du polypeptide recombinant en tant qu'agent antibactérien *in vitro* selon la revendication 16,
dans lesquelles il comprend en outre un lieur peptidique, liant le domaine catalytique de LytM et le domaine de liaison à la paroi cellulaire de lysostaphine,
dans lesquelles le lieur peptidique comprend de préférence la séquence d'acides aminés représentée dans SEQ ID NO : 3 ou SEQ ID NO : 4, de préférence, le lieur peptidique est constitué de la séquence SEQ ID NO : 3.

20. Polypeptide recombinant destiné à être utilisé selon la revendication 1, composition pharmaceutique ou vétérinaire comprenant le polypeptide recombinant destiné à être utilisé selon la revendication 5, utilisation du polypeptide recombinant selon la revendication 7, composition cosmétique ou de soin à usage cosmétique, de soin et hygiénique chez l'homme ou l'animal selon la revendication 10, polypeptide recombinant destiné à être utilisé dans le traitement de la mammite ou la prévention du développement de la mammite selon la revendication 12, utilisation du polypeptide recombinant en tant qu'agent antibactérien *in vitro* selon la revendication 16,
dans lesquelles le polypeptide recombinant comprend la séquence SEQ ID NO : 5, ou est codé par une séquence nucléotidique comprenant SEQ ID NO : 6 ;
ou le polypeptide recombinant comprenant la séquence SEQ ID NO : 7, ou est codé par une séquence nucléotidique comprenant SEQ ID NO : 8.

21. Polypeptide recombinant destiné à être utilisé selon la revendication 1, composition pharmaceutique ou vétérinaire comprenant le polypeptide recombinant destiné à être utilisé selon la revendication 5, utilisation du polypeptide recombinant selon la revendication 7, composition cosmétique ou de soin à usage cosmétique, de soin et hygiénique chez l'homme ou l'animal selon la revendication 10, polypeptide recombinant destiné à être utilisé dans le traitement de la mammite ou la prévention du développement de la mammite selon la revendication 12, utilisation du polypeptide recombinant en tant qu'agent antibactérien *in vitro* selon la revendication 16,
dans lesquelles la bactérie du genre *Staphylococcus* est *Staphylococcus aureus.*
